# EUROPEAN PATENT SPECIFICATION

(11) **EP 0 595 364 B1**
(45) Date of publication and mention of the grant of the patent: **24.07.1996**
(21) Application number: 93117721.6
(22) Date of filing: 02.11.1993
(51) Int. Cl.: C07C 251/58, C07C 271/60, C07D 295/08, C07D 213/74, C07D 239/34, C07D 303/12

(54) **Benz[e]indene derivatives and a process for preparing them as well as medicaments containing them and the use of them**
Benz[e]inden Derivate, Verfahren zu ihrer Herstellung sowie diese enthaltende Arzneimittel und ihre Verwendung
Dérivés de benz[e]indène, procédé pour leur préparation et médicaments qui leur contiennent, et leur utilisation

(30) Priority: 30.10.1992 HU 340692
(43) Date of publication of application: 04.05.1994
(73) Proprietor: EGIS GYOGYSZERGYAR RT., H-1106 Budapest (HU)
(72) Inventor: Reiter née Eszes, Klàra, H-1022 Budapest (HU); Budai, Zoltan, Dr., H-1025 Budapest (HU); Mezei, Tibor, Dr., H-1221 Budapest (HU); Blaskò, Gàbor, Dr., H-1113 Budapest (HU); Simig, Gyula, Dr., H-1126 Budapest (HU); Gyertyàn, Istvàn Dr., H-1165 Budapest (HU)
(74) Representative: Beszédes, Stephan G., Dr.

(56) References cited:
- FR-A- 2 177 930
- GB-A- 2 235 198
- FARMACO, EDIZIONE SCIENTIFICA vol. 30, no. 7 , July 1975 , PAVIA IT pages 568 - 80 G. SCAPINI ET. AL. 'Ricerche su Sostanze ad Attivita Antivirale'
- CHEMICAL ABSTRACTS, vol. 91, no. 17, 22 October 1979, Columbus, Ohio, US; abstract no. 140604d, P. ROVERI ET. AL. 'Synthesis of alpha-amino ketones in the benzo(e)indan series.' page 634 ;column 2 ;
- CHEMICAL ABSTRACTS, vol. 91, no. 17, 22 October 1979, Columbus, Ohio, US; abstract no. 140604d, P. ROVERI ET. AL. 'Synthesis of alpha-amino ketones in the benzo(e)indan series.' page 634 ;column 2 ; BOLL.CHIM.FARM, vol. 118, no.2, 1979 pages 102-4

## Description

The invention is concerned with novel benz[e]indene derivatives including their salts and quaternary ammonium derivatives with useful pharmaceutical, particularly tranquillo-sedative, anticonvulsive and antianginal, activities and a process for preparing them as well as medicaments containing these compounds and the use of them.

The structurally closest Prior Art compounds having the formula 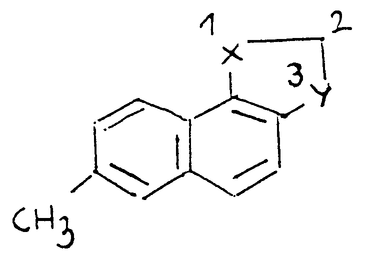
with the following meanings of X and Y

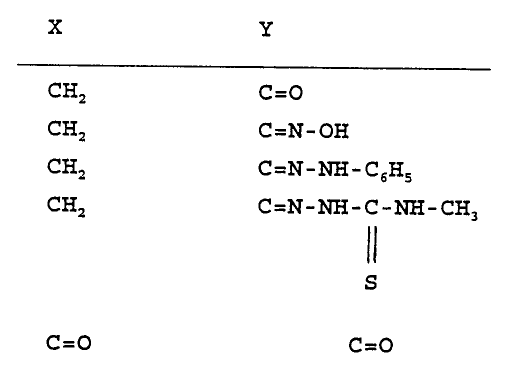

possess antiviral or antinflammatory effects [Il Farmaco-Ed. Sc. 30, 568-580 (1975); Arch. Pharm. (Weinheim) 316, 309-315 (1983)].

From French laying open print 2 177 930 there have been known 1,1-disubstituted indane-3-oxime ether and spiro-(cycloalkane-indane)-3-oxime ether having pharmacological effects on the central nervous system, particularly antireserpine effects, as well as antihistaminic and/or anticholinergic effects.

Moreover in Chemical Abstracts, Volume 91, No. 17, October 22, 1979, Abstract No. 140604d there has been described 5-methyl-benz[e]indane-1-oxime as intermediate.

Furthermore from GB-A-2,235,198 there have been known cyclopentenone oximes which are substituted on the oxime oxygen by an aminoalkylene group which is substituted by a hydroxy group. Tranquillo-sedative and antiangina as well as antiinflammatory activities have been indicated for these compounds; no mention has been of an anticonvulsive activity.

The problem underlying to the invention is to create novel benz[e]indene derivatives having valuable pharmacological properties, particularly tranquillo-sedative, anticonvulsive and antianginal, activities, a process for preparing them as well as medicaments containing these compounds and the use of them.

Surprisingly this has been solved by the invention.

Thus a subject-matter of the invention are benz[e]indene derivatives of the general formula 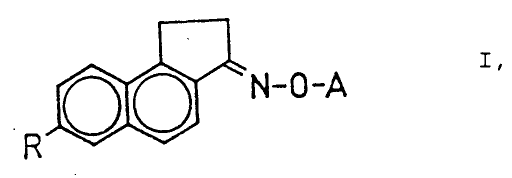
wherein
A represents a group of the formula 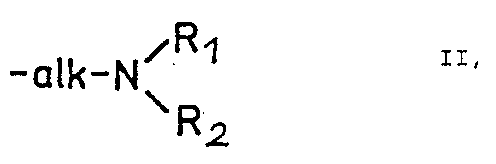
in which latter
- alk: means a C₂₋₇ alkylene group, optionally substituted by a hydroxy group and
- R₁ and R₂: stand independently for hydrogen or a C₁₋₇ alkyl, C₂₋₇ alkenyl, C₂₋₇ alkynyl, C₃₋₇ cycloalkyl, mono-(C₁₋₇)alkylamino-(C₁₋₇)alkyl or di-(C₁₋₇)alkylamino-(C₁₋₇)alkyl group or
- R₁ and R₂: together with the nitrogen atom to which they are attached form a 4 to 7 membered heterocyclic ring, optionally comprising an oxygen atom or a further nitrogen atom, which latter may be substituted by a phenyl, benzyl, pyridyl, pyrimidinyl or C₁₋₃ alkyl group which latter ones, in turn, may be substituted by a hydroxy or methoxy group or a halogen atom or a halophenyl group or
- R₁ and R₂: together with the nitrogen atom to which they are attached form a phthalimido group
or
A means a pyrimidino group, a 2,3-(epoxy)-propyl group or a group of the formula 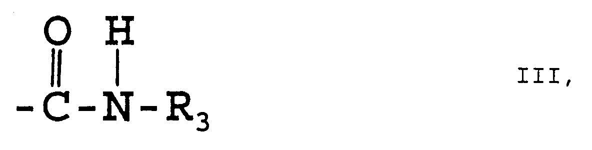
in which latter
- R₃: stands for a C₁₋₇ alkyl, C₂₋₇ alkenyl or C₃₋₈ cycloalkyl group
and
R denotes hydrogen or a C₁₋₇ alkyl group,
and their stereoisomers and optically active isomers and mixtures thereof, as well as acid addition salts and quaternary ammonium derivatives of these compounds.

Contrary to the compounds of French laying-open print 2 177 930 the compounds according to the invention are benz[e] indene-3-oxime ethers, i. e. a heterocyclic part is indene to which it has been condensed a benzene ring.

Contrary to Chemical Abstracts, Volume 91, No. 17, October 22, 1979, Abstract No. 140604d the compounds according to the invention are benz[e]indene-3-oxime ethers, i. e. a heterocyclic part is indane and the oxime group is etherified.

Contrary to GB-A-2,235,198 the compounds according to the invention contain a naphthalene ring fused to the cyclopentene ring. Additionally they have anticonvulsive activity.

The term "alkyl group" used throughout the text means straight or branched chained saturated aliphatic hydrocarbon groups having the given number of carbon atom(s), e.g. methyl, ethyl, propyl, isopropyl, n-butyl and tert-butyl groups. The term "alkenyl group" means straight or branched chained alkenyl groups containing the given number of carbon atoms, e.g. vinyl, allyl, 2-methylallyl, 1-propenyl, 1-butenyl, 2-butenyl and 2-hexenyl groups. The term "alkynyl group" covers straight or branched chained aliphatic hydrocarbon groups comprising at least one triple bond, e.g. the propargyl group. Examples of the cycloalkyl groups covered by the term "C₃₋₇ cycloalkyl" are cyclopropyl, cyclobutyl, cyclopentyl and cyclohexyl groups. As "4 to 7 membered rings" heteroaromatic or partially or completely saturated heterocyclic rings which contain as heteroatom a nitrogen atom and optionally an oxygen atom or a further nitrogen atom, e.g. piperidyl, morpholinyl, piperazinyl, imidazolyl, pyrimidinyl, pyrazolyl, imidazolinyl and pyrrolidinyl rings, are comprised. The term "halogen atom" encompasses all the four halogen atoms (fluorine, chlorine, bromine and iodine).

Preferably the alkyl group(s) which may be represented by R₁ and/or R₂ and/or R₃ and/or R including those occuring in mono-(C₁₋₇)alkylamino-(C₁₋₇)alkyl or di-(C₁₋₇)alkylamino-(C₁₋₇)alkyl groups which may be represented by R₁ and/or R₂ is/are such having from 1 to 4, particularly 1 to 3 or 6 carbon atom(s) and/or the alkyl group which may be carried by the heterocyclic ring which may be formed by R₁ and R₂ together with the nitrogen atom to which they are attached is such having from 1 or 2, particularly 1, carbon atom(s).

Particularly preferred alkyl groups which may be represented by R₁ and/or R₂ are methyl, [1-(methyl)-ethyl]-propyl and ethyl groups. A particularly preferred alkyl group which may be represented by R is the methyl group. Particularly preferred alkyl groups which may be carried by the heterocyclic ring which may be formed by R₁ and R₂ together with the nitrogen atom to which they are attached are methyl and ethyl groups.

It is also preferred that the alkenyl group(s) which may be represented by R₁ and/or R₂ or R₃ and/or the alkylene group which may be represented by alk is/are such having from 2 to 4, particularly 2 or 3, most particularly 3, carbon atoms and/or the alkynyl group(s) which may be represented by R₁ and/or R₂ is/are such having from 2 to 5, particularly 4 or 5, carbon atoms and/or the cycloalkyl group(s) which may be represented by R₁ and/or R₂ is/are such having from 3 to 6, particularly 3 or 6, carbon atoms and/or the cycloalkyl group which may be represented by R₃ is such having from 5 or 6, particularly 6, carbon atoms.

A particularly preferred alkenyl group is the allyl group. Particularly preferred alkylene groups are the ethylene and propylene groups. A particularly preferred alkynyl group is the 1,1-Di-(methyl)-propyn-2-yl group.

Furthermore it is preferred that the heterocyclic ring which may be formed by R₁ and R₂ together with the nitrogen atom to which they are attached is such having from 5 to 7, particularly 5 or 6, members, most particularly a piperidyl, piperazinyl, pyrinidinyl, morpholinyl, imidazolyl, pyrazolyl, imidazolinyl or pyrrolidinyl ring.

Moreover it is preferred that the halogen atom or the halogen atom of the halophenyl group by which the phenyl, benzyl, pyrimidinyl or alkyl group which may be carried by the heterocyclic ring which may be formed by R₁ and R₂ together with the nitrogen atom to which they are attached may be substituted is chlorine or fluorine, particularly the former one.

Particularly preferred benz[e]indene derivatives according to the invention are 3-[3'-(cyclopropylamino)-2'-(hydroxy)-propoxyimino]-2,3-di-[hydro]-1H-benz[e]indene and its acid addition salts and quaternary ammonium derivatives.

According to a further aspect of the present invention there is provided for a process for preparing the benz[e]indene derivatives according to the invention, which is characterized in that in a manner known per se
a) for preparing benz[e]indene derivatives of the formula I, wherein A stands for a 2,3-(epoxy)-propyl group reacting benz[e]indene derivatives of the formula 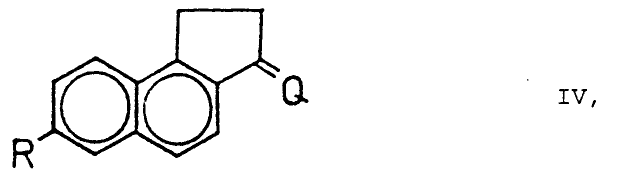 wherein Q represents a group of the formula =N-OH and R is as defined above, with halo-epoxypropyl compounds of the formula 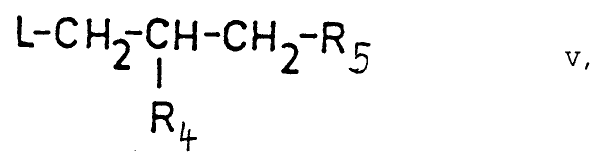 wherein L stands for halogen and R₄ and R₅ together represent oxygen, in the presence of basic condensing agents or
b) for preparing benz[e]indene derivatives of the formula I, wherein A denotes a group of the formula II, wherein alk, R₁ and R₂ are as defined above,
   b₁) reacting benz[e]indene derivatives of the formula IV, wherein Q stands for a group of the formula =N-OH and R is as defined above, or acid addition salts thereof with halo compounds of the formula 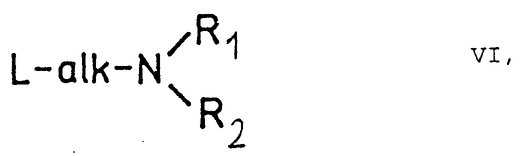 wherein alk, R₁ and R₂ are as defined above and L stands for halogen, or with acid addition salts thereof in the presence of basic condensing agents or
   b₂) reacting benz[e]indene derivatives of the formula I, wherein A stands for a 2,3-(epoxy)-propyl group, with amines of the formula 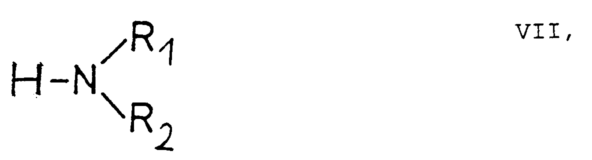 wherein R₁ and R₂ are as defined above, or
   b₃) reacting benz[e]indene derivatives of the formula IV, wherein Q stands for oxygen or sulphur and R is as stated above, with, optionally substituted, aminoalkyl halogenides of the formula VI, wherein alk, R₁ and R₂ are as defined above and L represents a group of the formula H₂N-0-, or with acid addition salts thereof in the presence of basic condensing agents or
c) for preparing benz[e]indene derivatives of the formula I, wherein A stands for a pyrimidino group reacting benz[e]indene derivatives of the formula IV, wherein Q represents a group of the formula =N-OH and R is as defined above, with halopyrimidines of the formula 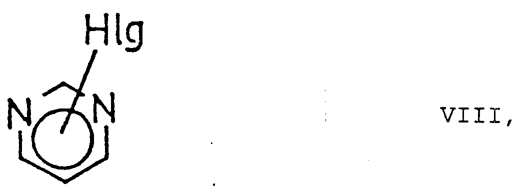 wherein Hlg represents halogen, in the presence of basic condensing agents or
d) for preparing benz[e]indene derivatives of the formula I, wherein A stands for a group of formula III, reacting benz[e]indene derivatives of the formula IV, wherein Q is a group of the formula =N-OH and R is as defined above, with isocyanates of the formula

   R₃-N=C=0 IX,

   wherein R₃ is as defined above,
and, if desired, converting benz(e)indene derivatives of the formula I thus obtained into acid addition salts or quaternary ammonium derivatives or, if desired, liberating free benz[e]indene derivative bases of the formula I from salts thereof and, if desired, converting the free benz[e]indene derivative bases into other acid addition salts or quaternary ammonium derivatives and/or, if desired, separating the stereoisomers and/or optically active isomers or racemizing the latter ones.

In variant a) of the process of invention as basic condensing agents in the presence of which the reaction can be carried out preferably alkali metal hydrides or alkali metal amides or mixtures thereof are used. It is particularly preferred to use the respective sodium compounds, but potassium hydride or potassium amide can also be applied. Suitably the reaction is carried out in inert aprotic solvents, particularly in dipolar aprotic and/or apolar aprotic solvents, such as dimethyl formamide, dimethyl sulfoxide, acetonitrile, acetone or benzene or homologues thereof or mixtures of such solvents. Advantageously reaction temperatures of from 0°C to 120°C are applied it being preferred to carry out the reaction at temperatures from 40°C to 50°C.

In variant b₁) of the process of invention as basic condensing agents in the presence of which the reaction can be carried out preferably alkali metal hydrides, alkali metal amides, alkali metal hydroxides or mixtures thereof are used. If alkali metal hydrides or amides are applied, suitably the reaction is carried out in aprotic solvents, preferably dipolar aprotic and/or apolar aprotic solvents, e.g. dimethyl formamide, dimethyl sulfoxide, acetonitrile, acetone or benzene or homologues thereof or mixtures of such solvents. If alkali metal hydroxides are used as basic condensing agents, suitably the reaction is carried out in protic and/or dipolar aprotic solvents, preferably in water, aliphatic alcohols, dimethyl formamide or dimethyl sulfoxide or mixtures thereof. Advantageously reaction temperatures of from 0°C to 120°C are applied it being preferred to carry out the reaction at temperatures from 40°C to 50°C.

In variant b₂) of the process of the invention the reaction is suitably carried out in protic solvents, preferably in aliphatic alcohols. In this case preferably temperatures of from 0°C to 120°C, particularly 70°C to 100°C, are applied. However, the reaction can also be performed without using any solvent. In the latter case preferably the reaction is carried out in a closed vessel at elevated temperatures, particularly at 50°C to 100°C. The thus obtained benz[e]indene derivatives of the for mula I can be isolated from the reaction mixture by methods known per se, e.g. by distilling off the solvent and crystallizing the residue or subjecting it to fractional distillation in vacuo.

In variant b₃) of the process of invention as basic condensing agents in the presence of which the reaction can be carried out preferably organic bases, e.g. pyridine, piperidine and/or morpholine, are used. Advantageously the reaction is carried out in protic and/or dipolar aprotic solvents. As protic solvents preferably aliphatic alcohols and as dipolar aprotic solvents preferably dimethyl formamide and/or dimethyl acetamide is/are used. Advantageously the reaction is carried out at temperatures of from 0°C to 120°C, preferably 70°C to 100°C. The thus obtained benz[e]indene derivatives of the formula I can be isolated from the reaction mixture by methods known per se, e.g. by evaporating the solvent.

In variant c) of the process of invention as basic condensing agents in the presence of which the reaction can be carried out preferably alkali metal amides or alkali metal hydrides or mixtures thereof are used. Advantageously the reaction is carried out in inert solvents, preferably in ethers such as tetrahydrofurane or dibutyl ether, or benzene or a homologue thereof or mixtures of such solvents. Advantageously reaction temperatures of from 30°C to 140°C are applied it being preferred to carry out the reaction at temperatures of from 50°C to 100°C.

In variant d) of the process of the invention advantageously the reaction is carried out in apolar aprotic solvents, preferably in benzene or a homologue thereof or dichloromethane, 1,2-di-(chloro)-ethane, chloroform or in mixtures thereof. Advantageously reaction temperatures of from 0°C to 80°C are applied it being preferred to carry out the reaction at 15°C to 30°C. The thus obtained benz[e]indene derivatives of the formula I can be isolated from the reaction mixture by methods known per se, e.g. by evaporating the solvent.

The benz[e]indene derivatives of the formula IV, in which Q stands for a group of the formula =N-OH used as starting substances for the process according to the invention can be prepared by the methods described in J. Chem. Soc. 1952, 3605-3607 or ibid. 1958, 2437-2440. The benz[e]indene derivatives of the formula IV, in which Q represents oxygen or sulphur, can be prepared as described in J. Chem. Soc. 1958, 10800-10804.

The compounds of the formula VI used as starting substances for the process according to the invention can be prepared e.g. according to the method specified in the Hungarian patent specification No. 201,324 or in J. Pharm. Sci. 58, 138-141 [1969].

The amines of the formula VII, the isocyanates of the formula IX and the halopyrimidines of the formula VIII used as starting substances for the process according to the invention are commercial products or can be prepared by methods known per se.

As already mentioned the benz[e]indene derivatives according to the invention have useful pharmaceutical, particularly tranquillo-sedative, anticonvulsive and antianginal, activities. They also have analgesic, local anaesthetic and antiinflammatory activities. At the same time they are only slightly toxic.

Hence by the invention also there are provided for medicaments which are characterized by that they contain as active principle(s) at least one compound according to the invention, suitably in admixture with at least one inert solid and/or liquid pharmaceutical carrier and/or diluent and/or further auxiliary excipient.

The biological activity of the compounds according to the invention is shown by the following tests:

### I. Acute toxicity

Mice belonging to the NMRI strain (body weight 20-25 g, both male and female) were used, 6 to 10 animals for each dose. The test compound was administered orally in a volume of 20 ml/kg. The applied maximal dose was 1000 mg/kg. After the treatment the animals were observed for a period of 7 days. The mice were kept in a plastic cage at room temperature. The animals got tap water and standard mouse fodder ad libitum. The toxicity data were determined by the aid of the method of Litchfield and Wilcoxon [Litchfield, J.T., Wilcoxon, F.W.: J. Pharmacol. Exp. Ther., 96, 99 (1949)]. The results are summarized in Table 1.

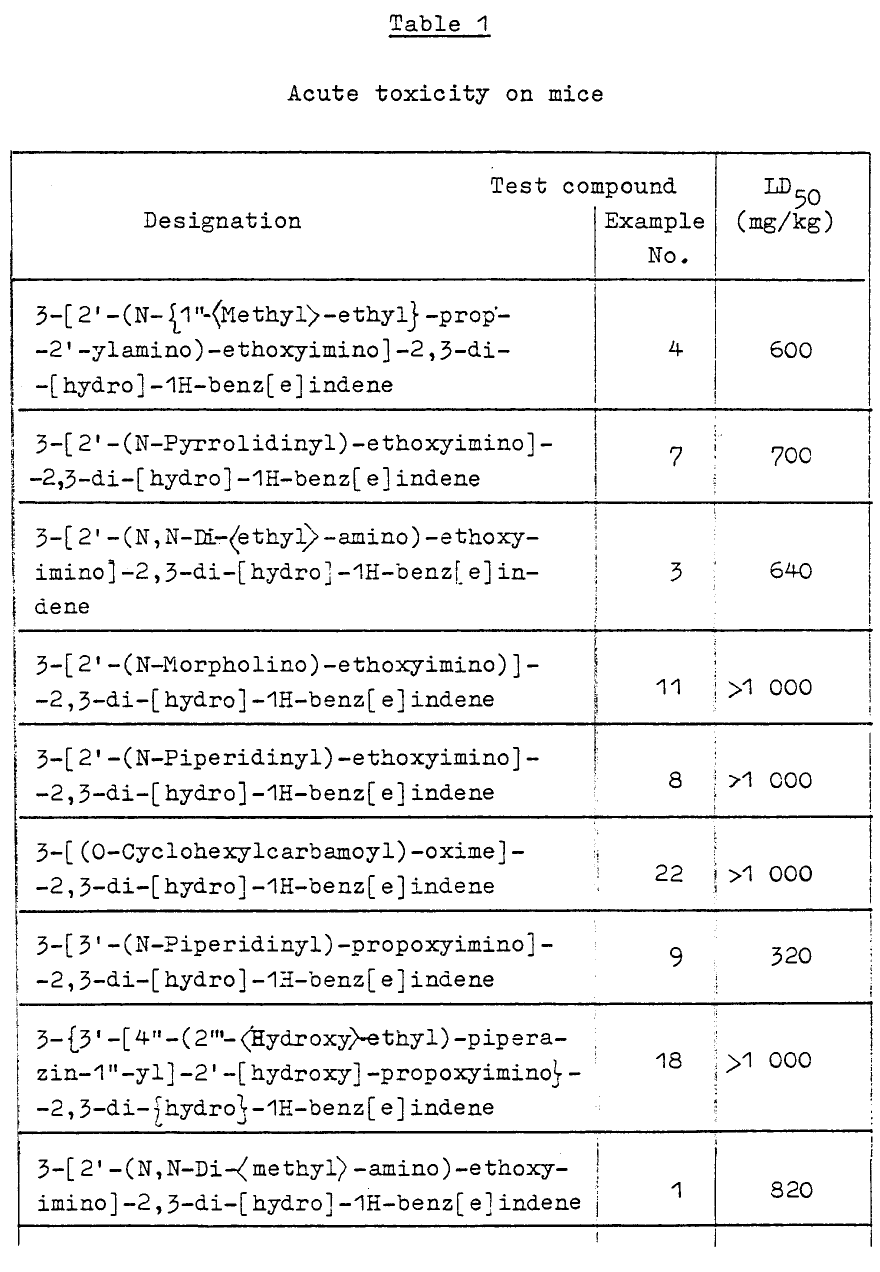

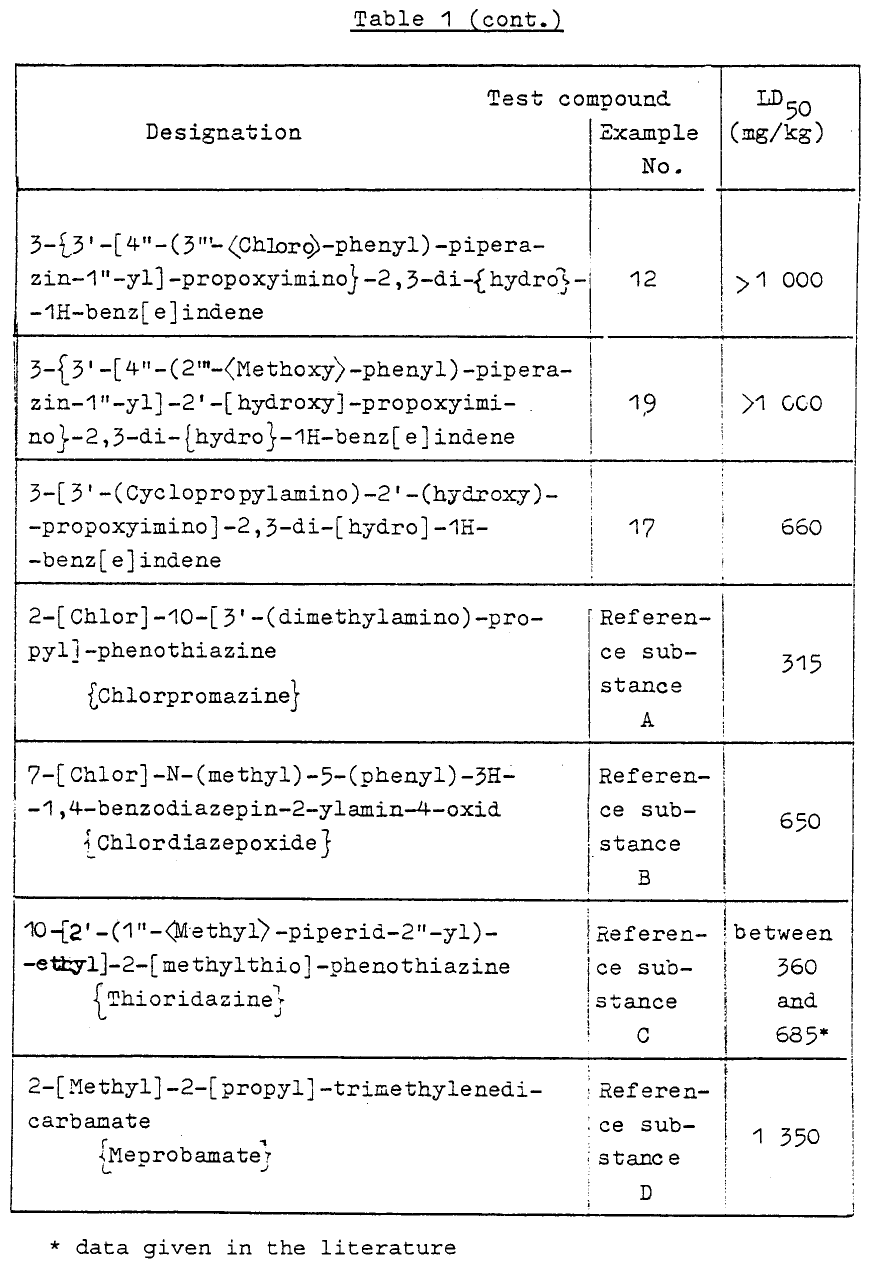

### II. Tranquillo-sedative effect

The hexobarbital narcosis potentiating effect was examined on mice. Groups consisting of 6 animals were used for each dose. The animals were treated orally with the test compound, sleeping having been induced 1 hour later by administering a 40 mg/kg i.v. dose of hexobarbital both to the test and control groups. The animals which had a sleeping time more than 2.5 times longer than the control group were considered to show a positive reaction. ED₅₀ values were calculated from the thus-transformed data [Kaergaard Nielson C. et al., Arch. Int. Pharmacodyn. 2, 170 (1967)]. The results are summarized in Table 2.

**Table 2**

| Hexobarbital narcosis on mice | | |
|---|---|---|
| Test compound | | ED₅₀ (mg/kg) (or effect observed in the given dose) |
| Designation | Example No. | |
| 3-[2'-(N,N-Di-〈methyl〉-amino)-ethoxyimino]-2,3-di-[hydro]-1H-benz[e]indene | 1 | 200 (100%) |
| 3-{3'-[4''-(3'''-〈Chloro〉-phenyl)-piperazin-1''-yl]-propoxyimino}-2,3-di-{hydro}-1H-benz[e]indene | 12 | 200 ( 83%) |
| 3-[2'-(N-Piperidinyl)-ethoxyimino]-2,3-di-[hydro]-1H-benz[e]indene | 8 | 100 ( 67%) |
| 3-{3'-[4''-(2'''-〈Methoxy〉-phenyl)-piperazin-1''-yl]-2'-[hydroxy]-propoxyimino}-2,3-di-{hydro}-1H-benz[e]indene | 19 | 67 |
| 3-[3'-(Cyclopropylamino)-2'-(hydroxy)-propoxyimino]-2,3-di-[hydro]-1H-benz[e]indene | 17 | 11 |
| 2-[Methyl]-2-[propyl]-trimethylenedicarbamate {Meprobamate} | Reference substance D | 260 |

From Table 2 it results that the compounds according to the invention have narcotic activities superior to that of the reference substance.

The spontaneous motility inhibiting activity was examined according to the method of Borsy et. al. Groups consisting of 3 mice each were treated orally with different doses of the compounds to be tested, then the test animals were placed in a Dews equipment. In this equipment the number of interruptions of infrared beam within 30 minutes was counted. From these data 50% inhibiting doses (ID₅₀) were determined by the aid of a line of regression. [Borsy, J., Csányi, E., Lázár, I.: Arch. Int. Pharmacodyn. 124, 1 (1960)]. The data are summarized in Table 3.

**Table 3**

| Motility inhibiting activity | | |
|---|---|---|
| Test compound | | ID₅₀ (mg/kg) |
| Designation | Example No. | |
| 3-{3'-[4''-(3'''-〈Chloro〉-phenyl)-piperazin-1''-yl]-propoxyimino}-2,3-di-{hydro}-1H-benz[e]indene | 12 | about 100 |
| 3-{3'-[4''-(2'''-〈Methoxy〉-phenyl)-piperazin-1''-yl]-2'-[hydroxy]-propoxyimino}-2,3-di-{hydro}-1H-benz[e]indene | 19 | 43 |
| 3-[3'-(Cyclopropylamino)-2'-(hydroxy)-propoxyimino]-2,3-di-[hydro]-1H-benz[e]indene | 17 | 6 |
| 2-[Methyl]-2-[propyl]-trimethylenedicarbamate {Meprobamate} | Reference substance D | 232 |

From Table 3 it results that the compounds according to the invention are superior to the reference substance as regards the motility inhibiting ativity.

The compound of Example 17, the most active member in the structural group, was examined in detail for tranquillizing and sedative effects to establish whether the supposed human therapeutic activity of the compound would be of antipsychotic or of anxiolytic character.

### III. A Antipsychotic effect

The antipsychotic (neuroleptic) effect was measured by the inhibition of the learned conditioned avoidance reflex. The male Wistar rats used for the study were of 120-150 g body weight at the commencement of learning. A shuttle box was used as experimental device; it consisted of two parts, 24x24.5x23 cm each, separated by a wall with a 6x9 cm gate. In response to a suitable warning stimulus, in order to avoid the punishing (unconditioned) stimulus, animals within the box passed through the gate from one part over the other. The warning, i.e. conditioned stimulus (CS), a white light (1 Hz) blinking for 15 seconds, was applied at the place of the actual animal existence. The unconditioned stimulus (US), in form of 0.6 mA intensity electric shock, was randomly applied to the paw during the last 5 seconds of the conditioned stimulus. Animal's movement during the CS and US from one part of the box over the other, was defined as avoidance and escape responses, respectively. Both responses ceased the actual stimulus and stopped the trial. The time elapsed until the next trial (intertrial interval, ITI) was 15 seconds. While one experiment was carried out daily, an experiment consisted of 80 trials. Learning efficiency was expressed as percentage of the successful to the total avoidances. Effect of the neuroleptic drugs was examined in animals with stabilized conditioned reflexes and with at least 75% learning efficiency. Dosing was carried out once a week, one hour before the measurement in the shuttle box. To calculate the neuroleptic effect (50% inhibiting dose, ID₅₀), results obtained after the treatment were compared to those obtained on the previous day (controls).

The extrapyramidal syndrome, the most important side effect limiting the application of the neuroleptic drugs in man, can be produced in experimental animals in the form of catalepsy. Our experiments in 150-160 g Wistar rats were performed according to Morpurgo. The catalepsy, appearing 60 minutes after dosing, was scored as follows. Both forelegs of each animal were put for 10 seconds onto a rubber stopper of 3 cm height, then for additional 10 seconds onto a same stopper of 9 cm height. 0.5 and 1 scores per foot (a total of maximum 3 scores) were given if animals failed to return their feet within 10 seconds from the lower and higher stoppers, respectively. The procedure was repeated in each 30 minutes during four hours, obtaining scores proportional to the degree of the catalepsy. Results were used to calculate the minimum effective dose [MED, the lowest dose resulting statistically significant alteration, c.f.: Morpurgo, C.: Arch. Int. Pharmacodyn., 137, 87 (1962)]. The data thus obtained are summarized in Table 4.

**Table 4**

| Antipsychotic effect on rat | | | | |
|---|---|---|---|---|
| Test compound | | Conditioned reflex ID₅₀ (mg/kg) | Catalepsy [MED (mg/kg)] | Reference ratio* |
| Designation | Example No. | | | |
| 3-[3'-(Cyclopropylamino)-2'-(hydroxy)-propoxyimino]-2,3-di-[hydro]-1H-benz[e]indene | 17 | 26.3 | 100 | 3.8 |
| 2-[Chlor]-10-[3'-(dimethylamino)-propyl]-phenothiazine {Chlorpromazine} | Reference substance A | 13.2 | 20 | 1.5 |
| 10-[2'-(1''-〈Methyl〉-piperid-2''-yl)-ethyl]-2-[methylthio]-phenothiazine {Thioridazine} | Reference substance C | 108 | 80 | 0.7 |

| | | | | |
|---|---|---|---|---|
| *Catalepsy MED | | | | |
| Conditioned reflex ID₅₀ | | | | |

From the data of the above Table 4 it can be seen that the compound 3-[3'-(cyclopropylamino)-2'-(hydroxy)-propoxyimino]-2,3-di-[hydro]-1H-benz[e]indene {Example 17}is superior to 10-[2'-(1''- methyl -piperid-2''-yl)-ethyl]-2-[methylthio]-phenothiazine {Thioridazine} and though inferior to 2-[chlor]-10-[3'-(dimethylamino)-propyl]-phenothiazine {Chlorpromazine} considering the antipsychotic effect it is superior to both reference substances in respect of the dose ratio of the corresponding side und main effects. Consequently a more favourable safety exists in patients treated with the compound 3-[3'-(cyclopropylamino)-2'-(hydroxy)-propoxyimino]-2,3-di-[hydro]-1H-benz[e]indene {Example 17}.

### III. B. Anxiolytic effect

The anxiolytic effect was tested by using the method of Vogel et. al. Male Wistar rats of 160 - 180 g body weight were kept free of food and drinking water for 24 and 48 hours, respectively. Test and carrier substances were administered intraperitoneally two hours before testing. Animals within the experimental chamber were provided with drinking water through an inserted tube. After the animals' each twenty lapping for water the device emitted a 2 mA intensity electric shock through the drinking tube. During 5 minutes the shocks tolerated by the animals in order to quench their thirst were counted. The effect of treatment was expressed as the % increase of the tolerated shocks. The minimum effective dose (MED) was determined for each test compound [Vogel, J.R., Beer, B., Clody, D.E.: Psychopharmacologia (Berl.) 21, 1 (1971)]. The data thus obtained are summarized in Table 5.

**Table 5**

| Anxiolytic effect on rat | | |
|---|---|---|
| Test compound | | MED (mg/kg) |
| Designation | Example No. | |
| 3-[3'-(Cyclopropylamino)-2'-(hydroxy)-propoxyimino]-2,3-di-[hydro]-1H-benz[e]indene | 17 | 0.1 |
| 7-(Chlor)-N-(methyl)-5-(phenyl)-3H-1,4-benzodiazepin-2-ylamin-4-oxid {Chlordiazepoxide} | Reference substance B | 2.5 |
| 2-[Methyl]-2-[propyl]-trimethylenedicarbamate {Meprobamate} | Reference substance D | 25 |

From the above Table 5 it can be seen that the compound 3-[3'-(cyclopropylamino)-2'-(hydroxy)-propoxyimino]-2,3-di-[hydro]-1H-benz[e]indene {Example 17} is superior to the reference substances by orders of magnitude.

Thus, it can be established that the compound 3-[3'-(cyclopropylamino)-2'-(hydroxy)-propoxyimino]-2,3-di-[hydro]-1H-benz[e]indene {Example 17} has, when administered in higher doses, a neuroleptic, while in case of smaller doses an anxiolytic character.

### IV. Anticonvulsive activity

The pentetrazole spasm inhibiting test was performed according to the modified method of Benziger and Hane. Groups of mice consisting of 6 animals each, belonging to the NMRI strain (body weight: 20-25 g), were treated orally with the compound to be tested and the vehicle without active agent, respectively. 1 hour after the treatment a dosage of 125 mg/kg of pentetrazole was administered to each animal, intraperitoneally, and the tonic spasm of the lower limb extensor was recorded [Benziger, R., Hane, D.: Arch. Int. Pharmacodyn., 167, 245 (1967)].

The inhibition of nicotine spasm and lethality was examined on mice according to the method of Stone. One hour after the oral treatment a dosage of 1.4 mg/kg of nicotine is injected intravenously, and the spasm developed as well as the lethality within 1 hour were recorded at both the test and control groups. [Stone, C.C., Mecklenburg, K.L., Torchiana, M.L.: Arch. Int. Pharmacodyn., 117, 419 (1958)].

The ED₅₀ values were determined according to the method of Litchfield and Wilcoxon. The results are shown in Table 6.

**Table 6**

| Anticonvulsive activity on mice | | | |
|---|---|---|---|
| Test compound | | Inhibition of pentetrazole spasm, ED₅₀ (mg/kg) | Inhibition of nicotine spasm, ED₅₀ (mg/kg) |
| Designation | Example No. | | |
| 3-[2'-(N-{1''-〈Methyl〉-ethyl}-prop-2'-ylamino)-ethoxyimino]-2,3-di-[hydro]-1H-benz[e]indene | 4 | - | 4 |
| 3-[2'-(N,N-Di-〈ethyl〉-amino)-ethoxyimino]-2,3-di-[hydro]-1H-benz[e]indene | 3 | 96 | 29 |
| 3-[2'-(N-Morpholino)-ethoxyimino)]-2,3-di-[hydro]-1H-benz[e]indene | 11 | 84 | - |
| 3-{3'-[4''-(3'''-〈Chloro〉-phenyl)-piperazin-1''-yl]-propoxyimino}-2,3-di-{hydro}-1H-benz[e]indene | 12 | 30 | - |
| 3,3,5-Tri-(methyl)-oxazolidin-2,4-dione {Trimethadione} | Reference substance E | 400 | - |
| α-(Cyclohexyl)-α-(2-piperidinoethyl)-benzylalcohol {Trihexyphenidyl} | Reference substance F | - | 20 |

From Table 6 it results that the compounds according to the invention have anticonvulsive activities superior to those of the reference substances or about equal to them.

### V. Analgesic effect

The test was carried out on mice, weighing 20-25 g, belonging to the NMRI strain according to the method of Newbould. 0.75% acetic acid is administered to the animals in a volume of 20 ml/kg one hour after the treatment with the test compounds and vehicles, respectively. The characteristic "writhing reactions" were counted for a period of 5 minutes, starting from the fifth minute after challenge. The number of writhing was observed for both the treated and the control group. Each group of animals consisted of at least 10 mice. The 50% inhibition doses (ID₅₀) were determined with the aid of a line of regression [Newbould, B.B.: Brit. J. Pharmacol., 35, 487 (1969)]. The results are disclosed in Table 7.

**Table 7**

| Analgesic effect on mice | | |
|---|---|---|
| Test compound | | ID₅₀ (mg/kg) |
| Designation | Example No. | |
| 3-{3'-[4''-(2'''-〈Methoxy〉-phenyl)-piperazin-1''-yl]-2'-[hydroxy]-propoxyimino}-2,3-di-{hydro}-1H-benz[e]indene | 19 | 65 |
| 3-[3'-(Cyclopropylamino)-2'-(hydroxy)-propoxyimino]-2,3-di-[hydro]-1H-benz[e]indene | 17 | 14 |
| Acetylsalicylic acid | Reference substance G | 261 |
| 4-(Hydroxy)-acetanilide {Paracetamol} | Reference substance H | 421 |

From Table 7 it results that the compounds according to the invention have analgesic activities superior to those of the reference substances.

### VI. Antianginal effect

The test was carried out on male rats weighing 180-220 g. The animals were narcotized with the aid of chloralose-urethane. ECG was registered by means of needle electrodes in standard II output. Antianginal effect was tested according to the method of Nieschultz. Experimental coronary insufficiency was induced by administering glanduitrine (4 NE/kg i.v.). The magnitude of the T-wave before and after the administration of glanduitrine was measured in the treated and control groups [Nieschultz, E., Popendiker, K., Hoffmann, I.: Arzneim.-Forsch., 5, 680 (1955)]. The results are summarized in Table 8

**Table 8**

| Antianginal effect on rat | | | |
|---|---|---|---|
| Test compound | | Inhibition in a dose of 2 mg/kg | ED₅₀ (mg/kg) |
| Designation | Example No. | | |
| 3-[2'-(N-{1''-〈Methyl〉-ethyl}-prop-2'-ylamino)-ethoxyimino]-2,3-di-[hydro]-1H-benz[e)indene | 4 | 100% | 0.19 |
| 3-[2'-(N-Morpholino)-ethoxyimino)]-2,3-di-[hydro]-1H-benz[e]indene | 11 | 54% | |
| 3-{3'-[4''-(3'''-〈Chloro〉-phenyl)-piperazin-1''-yl]-propoxyimino}-2,3-di-{hydro}-1H-benz[e]indene | 12 | 56% | |
| 3-[2'-(N-Piperidinyl)-ethoxyimino]-2,3-di-[hydro]-1H-benz[e]indene | 8 | 56% | |
| 3-[(0-Cyclohexylcarbamoyl)-oxime]-2,3-di-[hydro]-1H-benz[e]indene | 22 | 59% | |
| 3-{3'-[4''-(2'''-〈Hydroxy〉-ethyl)-piperazin-1''-yl]-2'-[hydroxy]-propoxyimino}-2,3-di-{hydro}-1H-benz[e]indene | 18 | 59% | |
| 3-[3'-(Cyclopropylamino)-2'-(hydroxy)-propoxyimino]-2,3-di-[hydro]-1H-benz[e]indene | 17 | 50% | |
| (RS)-N-[3,3-Di-(phenyl)-propyl]-α-methylphenethylamine {Prenylamine} | Reference substance I | 41% | 6.6 |

From Table 8 it results that the compounds according to the invention have antianginal activities superior to that of the reference substance.

### VII. Local analgesic effect

The test was carried out according to the method of Truant d'Amato. 0.2 ml of test material was injected around the nervus ischiadicus in the middle of femur with a needle of 1 cm length. The criterion of analgesic effect was the lack of the motoric control of foot muscles. The duration of effect was registered and the 50 percent effective concentration (EC₅₀) was calculated on the basis of the doseeffect curve. Lidocain was used as reference substance [Truant d'Amato, A.P., Wiedling, S.: Acta Chir. Scand., 116, 351 (1958)]. The results are disclosed in Table 9.

**Table 9**

| Local analgesic effect | | |
|---|---|---|
| Test compound | | EC₅₀ (%) |
| Designation | Example No. | |
| 3-[2'-(N-{1''-〈Methyl〉-ethyl}-prop-2'-ylamino)-ethoxyimino]-2,3-di-[hydro]-1H-benz[e]indene | 4 | 0.18 |
| 3-[2'-(N-Pyrrolidinyl)-ethoxyimino]-2,3-di-[hydro]-1H-benz[e]indene | 7 | 0.20 |
| 3-[3'-(N-Piperidinyl)-propoxyimino]-2,3-di-[hydro]-1H-benz[e]indene | 9 | 0.20 |
| 2-(Diethylamino)-2',6'-(aceto)-xylidide {Lidocain} | Reference substance K | 0.19 |

From Table 9 it results that the compounds according to the invention have about the same local analgesic activities as the reference substance.

### VIII. Antiinflammatory effect

The antiinflammatory effect was investigated on Wistar rats weighing 150-180g according to the method of Winter et al. 0.1 ml of a 1 per cent carrageen suspension was injected subcutaneously into the plantar region of one of the hind paws. The animals were fasted for 12 hours and received drinking water ad libitum. One hour before treatment with the test compound the rats were hydrated orally with 30 ml/kg of tap water. The test compounds and the vehicle were administered p.o. in a volume of 10 ml/kg, then 1 hour later carrageen was applied. The volume of the treated paw was measured by mercury-plethysmometer before and 3 hours after injection in such a way that displacement of the liquid arising from the volume alteration was indicated on a millimeter scale. The dose resulting in an inhibition of 30% (ID₃₀) was determined by the aid of a line of regression [Winter, C.A., Risley, E.A., Nuss, G.W.: Proc. Soc. Exp. Biol. Med., 111, 544 (1962)]. The results are summarized in Table 10.

**Table 10**

| Antiinflammatory effect on rat | | |
|---|---|---|
| Test compound | | ID₃₀ (mg/kg) |
| Designation | Example No. | |
| 3-[3'-(Cyclopropylamino)-2'-(hydroxy)-propoxyimino]-2,3-di-[hydro]-1H-benz[e]indene | 17 | 40 |
| Acetylsalicylic acid | Reference substance G | 62 |
| 4-(Hydroxy)-acetanilide {Paracetamol} | Reference substance H | 195 |

From Table 10 it results that the compound according to the invention has an antiinflammatory activity superior to the antiinflammatory activities of the reference substances.

The medicaments according to the invention can be in the form of pharmaceutical preparations. These contain beyond 1 or more benz[e]indene derivatives according to the invention as active principle(s) 1 or more inert solid and/or liquid pharmaceutical carrier(s) and/or diluent(s) and/or further auxiliary excipient(s).

The medicaments of the present invention can be prepared by methods known per se by admixing the active principle(s) with the inert solid and/or liquid carrier(s) and/or diluent(s) and/or further auxiliary excipient(s) and bringing the mixture to galenic form.

According to a further aspect of the present invention there is provided for the use of the compounds according to the invention for the preparation of medicaments, particularly such having tranquillo-sedative, anticonvulsive and antianginal effects.

The medicaments according to the invention can be used by administering to the patient an effective amount of the compound(s) according to the invention.

The invention is further illustrated by the following Examples the ratios of the components of the mixed solvents in all Examples being by volume.

### Example 1

### 3-[2'-(N,N-Di-〈methyl〉-amino)-ethoxyimino]-2,3-di-[hydro]-1H-benz[e]indene

a) 2,3-Dihydro-1H-benz[e]inden-3-one oxime (19.72 g, 0.1 mole) is converted into a salt in a saturated (30-40% by weight) aqueous solution of an alkali hydroxide (sodium hydroxide and/or potassium hydroxide) in the presence of dimethyl sulfoxide, and the salt thus obtained is reacted with 2-chloro-N,N-dimethylethylamine hydrochloride (15.85 g, 0.11 mole) at a temperature of 40-5O °C. The stirring is continued until the starting oxime cannot be detected in the reaction mixture by thin layer chromatography (Kieselgel 60 F₂₅₄, ethanol:ammonium hydroxide = 9:1). The reaction mixture is poured onto 600 g of icy water, the product is extracted with 400 cm³ of benzene, the organic phase is washed with saturated aqueous sodium chloride solution and dried over anhydrous magnesium sulfate. The solvent is distilled off under reduced pressure and the product thus obtained is purified by extraction with n-hexane.
Yield: 19.03 g (70.9%) of oil
Hydrochloride (1/1), m.p.: 237-241 °C.

| Analysis for the formula C₁₇H₂₁ClN₂0 (304.83): | | | | |
|---|---|---|---|---|
| Calculated: | C%=66.98, | H%=6.94, | Cl%=11.63, | N%=9.19; |
| Found: | C%=66.81, | H%=6.92, | Cl%=11.56, | N%=9.20. |

- UV: λₘₐₓ=: 242 nm (ε = 33922)
250 nm (ε = 44506)
260 nm (ε = 39153).

### Example 2

### 3-[3'-(N,N-Di-〈methyl〉-amino)-propoxyimino)-2,3-di-[hydro]-1H-benz[e]indene

One proceeds as specified in Example 1 except that instead of 2-chloro-N,N-dimethylethylamine hydrochloride 3-chloro-N,N-dimethylpropylamine hydrochloride (17.4 g, 0.11 mole) is used.
Yield: 22.42 g (74.9%) of oil
Hydrochloride (1/1), m.p.: 226-228 °C.

| Analysis for the formula C₁₈H₂₃ClN₂0 (318.84): | | | | |
|---|---|---|---|---|
| Calculated: | C%=67.80, | H%=7.27, | Cl%=11.12, | N%=8.78; |
| Found: | C%=67.69, | H%=7.14, | Cl%=11.15, | N%=8.73. |

- UV: λₘₐₓ=: 253 nm (ε = 43799)
262 nm (ε = 38229).

### Example 3

### 3-[2'-(N,N-Di ethyl -amino)-ethoxyimino]-2,3-di-[hydro]-1H-benz[e]indene

One proceeds as specified in Example 1 except that instead of 2-chloro-N,N-dimethylethylamine hydrochloride 2-chloro-N,N-diethylethylamine hydrochloride (18.93 g, 0.11 mole) is used.
Yield: 23.21 g (78.3%) of oil
Hydrochloride (1/1) M.p.: 207-211 °C.

| Analysis for the formula C₁₉H₂₅ClN₂0 (332.87): | | | | |
|---|---|---|---|---|
| Calculated: | C%=68.55, | H%=7.57, | Cl%=10.65, | N%=8.42; |
| Found: | C%=68.43, | H%=7.65, | Cl%=10.73, | N%=8.35. |

- UV: λₘₐₓ=: 242 nm (ε = 33124)
254 nm (ε = 48731)
262 nm (ε = 38844).

### Example 4

### 3-[2'-(N-{1''-〈Methyl〉-ethyl}-prop-2'-ylamino)-ethoxyimino]-2,3-di-[hydro]-1H-benz[e]indene

One proceeds as specified in Example 1 except that instead of 2-chloro-N,N-dimethylethylamine hydrochloride N-(2-chloroethyl)-N-(1-methylethyl)-prop-2-ylamine hydrochloride (22.02 g, 0.11 mole) is used.
Yield: 24.82 g (76.5%) of oil
Hydrochloride (1/1), m.p.: 190-197 °C.

| Analysis for the formula C₂₁H₂₉ClN₂0 (360.92): | | | | |
|---|---|---|---|---|
| Calculated: | C%=69.88, | H%=8.10, | Cl%= 9.82, | N%=7.76; |
| Found: | C%=69.72, | H%=8.24, | Cl%=10.06, | N%=7.61. |

- UV: λₘₐₓ=: 242 nm (ε = 32826)
253 nm (ε = 45125)
261 nm (ε = 40103).

### Example 5

### (±)-3-[2'-(N,N-Di- methyl -amino)-2'-(methyl)-ethoxyimino]-2,3-di-[hydro]-1H-benz[e]indene

One proceeds as specified in Example 1 except that instead of 2-chloro-N,N-dimethylethylamine hydrochloride 1-chloro-N,N-dimethyl-prop-2-ylamine (13.38 g, 0.11 mole) is used.
Yield: 24.15 g (84.9%) of oil
Hydrochloride (1/1), m.p.: 234-236 °C.

| Analysis for the formula C₁₈H₂₃ClN₂0 (318.84): | | | | |
|---|---|---|---|---|
| Calculated: | C%=67.80, | H%=7.23, | Cl%=11.12, | N%=8.79; |
| Found: | C%=67.74, | H%=7.18, | Cl%=11.23, | N%=8.67. |

- UV: λₘₐₓ=: 243 nm (ε = 35307)
252 nm (ε = 45796)
260 nm (ε = 40066).

### Example 6

### 3-[3'-(2'',N,N-Tri-〈methyl〉-amino)-propoxyimino]-2,3-di-[hydro]-1H-benz[e]indene

One proceeds as specified in Example 1 except that instead of 2-chloro-N,N-dimethylethylamine hydrochloride 3-chloro-2,N,N-trimethylpropylamine (14.92 g, 0.11 mole) is used.
Yield: 27.57 g (93.0%) of oil
Hydrochloride (1/1) M.p.: 194-196 °C (isopropanol).

| Analysis for the formula C₁₉H₂₅ClN₂0 (332.822): | | | | |
|---|---|---|---|---|
| Calculated: | C%=68.56, | H%=7.57, | Cl%=10.65, | N%=8.41; |
| Found: | C%=68.41, | H%=7.45, | Cl%=10.68, | N%=8.36. |

- UV: λₘₐₓ=: 243 nm (ε = 33070)
253 nm (ε = 42544)
262 nm (ε = 36457).

### Example 7

### 3-[2'-(N-Pyrrolidinyl)-ethoxyimino]-2,3-di-[hydro]-1H-benz[e]indene

One proceeds as specified in Example 1 except that instead of 2-chloro-N,N-dimethylethylamine hydrochloride N-(2-chloroethyl)-pyrrolidine hydrochloride (18.71 g, 0.11 mole) is used.
Yield: 20.95 g (71.2%), m.p.: 89-92°C (petrol)
Hydrochloride/ethanol (1/1/1) {the molar ratio of the title compound : hydrochloride : ethanol of the solvate with ethanol}, M.p.: 219-227°C (ethanol).

| Analysis for the formula C₂₁H₂₉ClN₂0₂ (376.94): | | | | |
|---|---|---|---|---|
| Calculated: | C%=66.92, | H%=7.76, | Cl%=9.41, | N%=7.43; |
| Found: | C%=66.90, | H%=7.56, | Cl%=9.37, | N%=7.40. |

- UV: λₘₐₓ=: 252 nm (ε = 47042)
260 nm (ε = 41210).

### Example 8

### 3-[2'-(N-Piperidinyl)-ethoxyimino]-2,3-di-[hydro]-1H-benz[e]indene

One proceeds as specified in Example 1 except that instead of 2-chloro-N,N-dimethylethylamine hydrochloride N-(2-chloroethyl)-piperidine hydrochloride (20.25 g, 0.11 mole) is used.
Yield: 27.14 g (88.0%) of oil
Hydrochloride (1/1) M.p.: 209-214 °C (isopropanol).

| Analysis for the formula C₂₀H₂₅ClN₂0 (344.89): | | | | |
|---|---|---|---|---|
| Calculated: | C%=69.65, | H%=7.31, | Cl%=10.28, | N%=8.12; |
| Found: | C%=69.70, | H%=7.28, | Cl%=10.22, | N%=8.02. |

- UV: λₘₐₓ=: 244 nm (ε = 34500)
252 nm (ε = 43988)
260 nm (ε = 37981).

### Example 9

### 3-[3'-(N-Piperidinyl)-propoxyimino]-2,3-di-[hydro]-1H-benz[e]indene

One proceeds as specified in Example 1 except that instead of 2-chloro-N,N-dimethylethylamine hydrochloride N-(3-chloropropyl)-piperidine hydrochloride (21.8 g, 0.11 mole) is used.
Yield: 26.28 g (81.5%)
Hydrochloride (1/1) M.p.: 205-208 °C.

| Analysis for the formula C₂₁H₂₇ClN₂0 (358.93): | | | | |
|---|---|---|---|---|
| Calculated: | C%=70.27, | H%=7.58, | Cl%=9.89, | N%=7.80; |
| Found: | C%=70.30, | H%=7.55, | Cl%=9.90, | N%=7.74. |

- UV: λₘₐₓ=: 253 nm (ε = 37950)
262 nm (ε = 36284)
280 nm (ε = 12820).

### Example 10

### 3-[2'-(Hexahydro-1H-azepinyl)-ethoxyimino]-2,3-di-[hydro]-1H-benz[e]indene

One proceeds as specified in Example 1 except that instead of 2-chloro-N,N-dimethylethylamine hydrochloride 1-(2-chloro-ethyl)-hexahydro-1H-azepine hydrochloride (21.8 g, 0.11 mole) is used.
Yield: 26.42 g (81.3%), m.p.: 46-47 °C
Hydrochloride (1/1) M.p.: 208-219 °C (ethanol).

| Analysis for the formula C₂₁H₂₇ClN₂0 (358.920): | | | | |
|---|---|---|---|---|
| Calculated: | C%=70.27, | H%=7.58, | Cl%=9.88, | N%=7.81; |
| Found: | C%=70.30, | H%=7.64, | Cl%=9.76, | N%=7.80. |

- UV: λₘₐₓ=: 243 nm (ε = 34766)
253 nm (ε = 46986)
261 nm (ε = 41378).

### Example 11

### 3-[2'-(N-Morpholino)-ethoxyimino)]-2,3-di-[hydro]-1H-benz[e]indene

One proceeds as specified in Example 1 except that instead of 2-chloro-N,N-dimethylethylamine hydrochloride 4-(2-chloroethyl)-morpholine hydrochloride (20.47 g, 0.11 mole) is used.
Yield: 24.77 g (79.8%)
Hydrochloride (1/1), m.p.: 197-216 °C.

| Analysis for the formula C₁₉H₂₃ClN₂0₂ (346.87): | | | | |
|---|---|---|---|---|
| Calculated: | C%=65.79, | H%=6.68, | Cl%=10.22, | N%=8.08; |
| Found: | C%=65.65, | H%=6.68, | Cl%=10.25, | N%=8.15. |

- UV: λₘₐₓ=: 242 nm (ε = 33503)
254 nm (ε = 43875)
262 nm (ε = 38288).

### Example 12

### 3-{3'-[4''-(3'''-〈Chloro〉-phenyl)-piperazin-1''-yl]-propoxyimino}-2,3-di-{hydro}-1H-benz[e]indene

One proceeds as specified in Example 1 except that instead of 2-chloro-N,N-dimethylethylamine hydrochloride 1-(3-chloropropyl)-4-(3-chlorophenyl)-piperazine hydrochloride (34.06 g, 0.11 mole) is used.
Yield: 34.46 g (79.4%), m.p:165-168 °C (acetone)
Hydrochloride (1/1), m.p.: 198-203 °C (ethanol)

| Analysis for the formula C₂₆H₂₉Cl₂N₃0 (470.45): | | | | |
|---|---|---|---|---|
| Calculated: | C%=66.38, | H%=6.22, | Cl%=15.07, | N%=8.93; |
| Found: | C%=66.42, | H%=6.18, | Cl%=15.11, | N%=8.90. |

- UV: λₘₐₓ=: 250 nm (ε = 69748)
260 nm (ε = 41948).

### Example 13

### 3-[3'-(N-Phthalimido)-propoxyimino]-2,3-di-[hydro]-1H-benz[e]indene

2,3-dihydro-1H-benz[e]indene-3-one oxime (19.72 g, 0.1 mole) is converted into a salt with sodium hydride (4.8 g, 0.1 mole) in dimethylformamide (50% oily dispersion), and the salt thus obtained is reacted with N-(3-bromopropyl)-phthalimide (29.49 g, 0.11 mole) at a temperature of 40 to 50°C. The stirring is continued until the starting oxime cannot be detected in the reaction mixture by thin layer chromatography (Kieselgel 60 F₂₅₄, ethanol: ammonium hydroxide = 9:1). Then ethanol is added to the mixture, it is diluted with water and the separated product is filtered off.
Yield: 30.29 g (78.8%), m.p.: 161-163°C (methylethylketone)

| Analysis for the formula C₂₄H₂₀N₂0₃ (384.44): | | | |
|---|---|---|---|
| Calculated: | C%=74.98, | H%=5.24, | N%=7.29; |
| Found: | C%=74.81, | H%=5.24, | N%=7.44. |

- UV: λₘₐₓ=: 219 nm (ε = 51786)
232 nm (ε = 39809)
240 nm (ε = 37521).

### Example 14

### 3-[Pyrimidin-2'-yloxyimino]-2,3-di-[hydro]-1-benz[e]indene

One proceeds as specified in Example 13 except that instead of N-(3-bromopropyl)-phthalimide 2-chloropyrimidine (12.60 g, 0.11 mole) is used.
Yield: 21.97 g (79.8%), m.p.: 176-178 °C (isopropanol).
(E)-2-butanedioate (2/1), m.p.: 190-195°C (ethanol).

| Analysis for the formula C₃₈H₃₀N₆0₆ (666.67): | | | |
|---|---|---|---|
| Calculated: | C%=68.45, | N%=12.61, | H%=4.53; |
| Found: | C%=68.47, | N%=12.58, | H%=4.47. |

- UV: λₘₐₓ=: 252 nm (ε = 100438)
262 nm (ε = 93834).

### Example 15

### 3-[1'-(2',3'-Epoxy)-propoxyimino]-2,3-di-[hydro]-1H-benz[e]indene

2,3-Dihydro-1H-benz[e]inden-3-one oxime (19.72 g, 0.1 moles) is converted into a salt with sodium hydroxide (4.8 g, 0.1 mole, 50% oily dispersion in dimethylformamide) and the salt thus obtained is reacted with 1,2-epoxy-3-chloropropane (10.17 g, 0.11 mole) at a temperature of 40 to 50°C. The stirring is continued until the starting oxime cannot be detected in the reaction mixture by thin layer chromatography (Kieselgel 60 F₂₅₄, ethanol:ammonium hydroxide = 9:1). Then ethanol is added to the mixture, it is diluted with water and the product thus obtained is extracted with benzene. The solvent is distilled off. The product thus obtained does not require further purification.
Yield: 21.61 g (85.3%), m.p.: 74-76°C (n-hexane).

| Analysis for the formula C₁₆H₁₅NO₂ (253.304): | | | |
|---|---|---|---|
| Calculated: | C%=75.87, | H%=5.97, | N%=5.53; |
| Found: | C%=75.79, | H%=5.97, | N%=5.51. |

- UV: λₘₐₓ=: 244 nm (ε = 32619)
253 nm (ε = 41745)
262 nm (ε = 35645).

### Example 16

### 3-{3'-[N-(1''-〈Methyl〉-ethyl)-prop-2'-ylamino]-2'-[hydroxy]-propoxyimino}-2,3-di-{hydro}-1H-benz[e]indene

a) One proceeds as specified in Example 15.
b) The product obtained according to Example 15 is reacted with N-(1-methylethyl)-prop-2-ylamine (9.51 g, 0.094 mole) in ethanol (21.65 g, 0.085 mole) at the boiling point of the mixture. The boiling is continued until the starting substance cannot be detected in the reaction mixture by thin layer chromatography (Kieselgel 60, F₂₅₄, ethanol:ammonium hydroxide = 9:1). The solvent is distilled off and the product is purified by acidic-alkaline precipitation.
Yield: 26.82 g (89%)
Hydrochloride/water (1/1/1), m.p.: 179-186°C (methylethylketone).

| Analysis for the formula C₂₂H₃₃ClN₂0₃ (408.98): | | | | |
|---|---|---|---|---|
| Calculated: | C%=64.61, | H%=8.13, | Cl%=8.67, | N%=6.85; |
| Found: | C%=64.57, | H%=8.11, | Cl%=8.69, | N%=6.94. |

- UV: λₘₐₓ=: 242 nm (ε = 33743)
253 nm (ε = 42625)
262 nm (ε = 36938),

### Example 17

### 3-[3'-(Cyclopropylamino)-2'-(hydroxy)-propoxyimino]-2,3-di-[hydro]-1H-benz[e]indene

a) One proceeds according to Example 15.
b) One proceeds according to point b) of Example 16 except that instead of N-(1-methylethyl)-prop-2-ylamine cyclopropylamine (5.37 g, 0.094 mole) is used.
Yield: 21.37 g (81%), m.p.: 86-87°C (n-hexane:ethyl acetate= =9:1).
Hydrochloride (1/1) m.p.: 167-176°C (isopropanol)

| Analysis for the formula C₁₉H₂₃ClN₂0₂ (346.87): | | | | |
|---|---|---|---|---|
| Calculated: | C%=65.79, | H%=6.68, | Cl%=10.22, | N%=8.08; |
| Found: | C%=65.71, | H%=6.65, | Cl%=10.22, | N%=8.00. |

- UV: λₘₐₓ=: 241 nm (ε = 30179)
252 nm (ε = 37285)
261 nm (ε = 33263).

### Example 18

### 3-{3'-[4''-(2'''-〈Hydroxy〉-ethyl)-piperazin-1''-yl]-2'-[hydroxy]-propoxyimino}-2,3-di-{hydro}-1H-benz[e]indene

a) One proceeds according to Example 15.
b) One proceeds according to point b) of Example 16 except that instead of N-(1-methylethyl)-prop-2-ylamine 1-(2-hydroxyethyl)-piperazine (12.24 g, 0.094 mole) is used.
Yield: 24.71 g (75.8%), m.p.: 104-107°C.
(Z)-2-butenedioate (1/2), m.p.: 183-187°C.

| Analysis for the formula C₃₀H₃₇N₃0₁₁ (615.65): | | | |
|---|---|---|---|
| Calculated: | C%=58.53, | H%=6.06, | N%=6.83; |
| Found: | C%=58.47, | H%=6.10, | N%=6.76. |

- UV: λₘₐₓ=: 214 nm (ε = 30800)
254 nm (ε = 39011)
263 nm (ε = 33671).

### Example 19

### 3-{3'-[4''-(2'''-〈Methoxy〉-phenyl)-piperazin-1''-yl]-2'-[hydroxy]-propoxyimino}-2,3-di-{hydro}-1H-benz[e]indene

a) One proceeds according to Example 15.
b) One proceeds according to point b) of Example 16 except that instead of N-(1-methylethyl)-prop-2-ylamine 1-(2-methoxyphenyl)-piperazine (21.15 g, 0.11 mole) is used.
Yield: 37.3 g (83.7%).
Hydrochloride (1/1), m.p.: 187-190 °C.

| Analysis for the formula C₂₇H₃₂ClN₃0₃ (482.03): | | | | |
|---|---|---|---|---|
| Calculated: | C%=67.27, | H%=6.69, | Cl%=7.36, | N%=8.72; |
| Found: | C%=67.21, | H%=6.63, | Cl%=7.37, | N%=8.68. |

- UV: λₘₐₓ=: 242 nm (ε = 37553)
252 nm (ε = 43057)
260 nm (ε = 36165).

### Example 20

### 3-{3'-[4''-(3'''-〈Chloro〉-phenyl)-piperazin-1''-yl]-2'-[hydroxy]-propoxyimino}-2,3-di-{hydro}-1H-benz[e]indene

a) One proceeds according to Example 15.
b) One proceeds according to point b) of Example 16 except that instead of N-(1-methylethyl)-prop-2-ylamlne 1-(3-chlorophenyl)-piperazine is used.
Yield: 41.63 g (92.5%), m.p.: 153-156°C.
(Z)-2-butenedioate (1/1), m.p.: 153-156°C.

| Analysis for the formula C₃₀H₃₂ClN₃0₆ (566.07): | | | | |
|---|---|---|---|---|
| Calculated: | C%=63.65, | H%=5.70, | Cl%=6.26, | N%=7.43; |
| Found: | C%=63.69, | H%=5.76, | Cl%=6.27, | N%=7.50. |

- UV: λₘₐₓ=: 244 nm (ε = 45139)
252 nm (ε = 52298)
262 nm (ε = 40837).

### Example 21

### 3-[(0-Allylcarbamoyl)-oxime]-2,3-di-[hydro]-lH-benz[e]indene

2,3-Dihydro-1H-benz[e]-inden-3-one oxime (19.72 g, 0.1 mole) is reacted with allyl isocyanate (9.14 g, 0.11 mole) in dichloromethane at a temperature between 16°C and 28°C. The stirring is continued until the starting oxime cannot be detected in the reaction mixture by thin layer chromatography.
Yield: 27.67 g (98.7%), m.p.: 161-166°C (isopropanol).

| Analysis for the formula C₁₇H₁₆N₂0₂ (280.33): | | | |
|---|---|---|---|
| Calculated: | C%=72.84, | H%=5.75, | N%=10.00; |
| Found: | C%=72.82, | H%=5,72, | N%= 9.66. |

- UV: λₘₐₓ=: 243 nm (ε = 36067)
250 nm (ε = 54527)
260 nm (ε = 55017).

### Example 22

### 3-[(0-Cyclohexylcarbamoyl)-oxime]-2,3-di-[hydro]-1H-benz[e]indene

One proceeds as specified in Example 21 except that instead of allyl isocyanate cyclohexyl isocyanate (13.77 g, 0.11 mole) is used.
Yield: 31.91 g (99%), m.p.: 176-184°C (isopropanol).

| Analysis for the formula C₂₀H₂₂N₂0₂ (322.4): | | | |
|---|---|---|---|
| Calculated: | C%=74.51, | H%=6.88, | N%=8.69; |
| Found: | C%=74.57, | H%=6.92, | N%=8.67. |

- UV: λₘₐₓ=: 244 nm (ε = 35713)
252 nm (ε = 54914)
260 nm (ε = 54564).

### Example 23

### 3-{3'-[Cyclohexylamino]-2'-[hydroxy]-propoxyimino}-2,3-di-{hydro}-1H-benz[e]indene

a) One proceeds according to Example 15.
b) One proceeds according to point b) of Example 16 except that instead of N-(1-methylethyl)-prop-2-ylamine cyclohexylamine (9.32 g, 0.094 mole) is used.
Yield: 23.97 g (80%), mp.: 131-132°C (ethanol).
Hydrochloride (1/1), m.p.: 204-211 °C (ethanol).

| Analysis for the formula C₂₂H₂₉ClN₂0₂: | | | | |
|---|---|---|---|---|
| Calculated: | C%=67.94, | H%=7.52, | Cl%=9.12, | N%=7.20; |
| Found: | C%=67.75, | H%=7.49, | Cl%=9.29, | N%=7.23. |

- UV: λₘₐₓ=: 244 nm (ε = 31696)
253 nm (ε = 40483)
262 nm (ε = 34411)
282 nm (ε = 13685)
301 nm (ε = 11670).

### Example 24

### 3-{3'-[4''-(4'''-〈Chloro〉-phenyl)-piperazin-1''-yl]-2'-[hydroxy]-propoxyimino}-2,3-di-{hydro}-1H-benz[e]indene

a) One proceeds according to Example 15.
b) One proceeds according to point b) of Example 16 except that instead of N-(1-methylethyl)-prop-2-ylamine 1-(4-chlorophenyl)-piperazine (18.49 g, 0.094 mole) is used.
Yield: 32.5 g (85.0%), m.p.: 155-159°C (toluene).
Hydrochloride (1/1), m.p.: 199-211 °C (ethanol).

| Analysis for the formula C₂₆H₂₉Cl₂N₃0₂ (486.46): | | | | |
|---|---|---|---|---|
| Calculated: | C%=64.19, | H%=6.01, | Cl%=14.58, | N%=8.64; |
| Found: | C%=67.23, | H%=6.13, | Cl%=14.41, | N%=8.99. |

- UV: λₘₐₓ=: 253 nm (ε = 55614)
262 nm (ε = 44063)
280 nm (ε = 15401)
290 nm (ε = 18310)
302 nm (ε = 14460).

### Example 25

### 3-{3'-[4''-(4'''-〈Fluoro〉-phenyl-methyl)-piperazin-1''-yl]-2'-[hydroxy]-propoxyimino}-2,3-di-{hydro}-1H-benz[e]indene

a) One proceeds according to Example 15.
b) One proceeds according to point b) of Example 16 except that instead of N-(1-methylethyl)-prop-2-ylamine 1-[4-(4'-〈fluoro〉-phenyl)-methyl]-piperazine (18.26 g, 0.094 mole) is used.
Yield: 34.58 g (91.0%), m.p.:114-116°C (ethanol).
Hydrochloride (1/2), m.p.: 207-213°C (ethanol).

| Analysis for the formula C₂₇H₃₂FCl₂N₃0₂ (520.49): | | | | | |
|---|---|---|---|---|---|
| Calculated: | C%=62.31, | H%=6.19, | F%=3.65, | Cl%=13.62, | N%=8.07; |
| Found: | C%=61.89, | H%=6.42, | F %= 3.53, | Cl%=13.62, | N%=8.12. |

- UV: λₘₐₓ=: 242 nm (ε = 31569)
253 nm (ε = 35082)
262 nm (ε = 35370)
290 nm (ε = 15891)
302 nm (ε = 12381).

### Example 26

### 3-{3'-[4''-(4'''-〈Chloro〉-phenyl-methyl)-piperazin-1'''-yl]-2'-[hydroxy]-propoxyimino}-2,3-di-{hydro}-1H-benz[e]indene

a) One proceeds according to Example 15.
b) One proceeds according to point b) of Example 16 except that instead of N-(1-methylethyl)-prop-2-ylamine 1-[4-(4'-〈chloro〉-phenyl-methyl]-piperazine (19.81 g, 0.094 mole) is used.
Yield: 36.48 g, m.p.: 138-140°C (ethanol).
Hydrochloride (1/2), m.p.: 207-214°C (ethanol).

| Analysis for the formula C₂₇H₃₂Cl₃N₃0₂ (536.95): | | | | |
|---|---|---|---|---|
| Calculated: | C%=60.39, | H%=6.01, | Cl%=19.81, | N%=7.83; |
| Found: | C%=60.33, | H%=6.01, | Cl%=19.57, | N%=7.83. |

- UV: λₘₐₓ=: 244 nm (ε = 31425)
263 nm (ε = 35082)
281 nm (ε = 13726)
292 nm (ε = 15911)
304 nm (ε = 12727).

### Example 27

### 3-{3'-[4''-(Pyrid-2'''-yl)-piperazin-1''-yl]-2'-[hydroxy]-propoxyimino}-2,3-di-{hydro}-1H-benz[e]indene

a) One proceeds according to Example 15.
b) One proceeds according to point b) of Example 16 except that instead of N-(1-methylethyl)-prop-2-ylamine 1-(2-pyridyl)-piperazine (15.34 g, 0.094 mole) is used.
Yield: 30.80 g (87%), n.p.: 138-140°C (ethanol).
Hydrochloride (1/2), m.p.: 172-175 °C (methanol).

| Analysis for the formula C₂₅H₃₀Cl₂N₄0₂ (489.46): | | | | |
|---|---|---|---|---|
| Calculated: | C%=61.35, | H%=6.18, | Cl%=14.49, | N%=11.45; |
| Found: | C%=59.58, | H%=6.09, | Cl%=14.14, | N%=11.00. |

- UV: λₘₐₓ=: 242 nm (ε = 44245)
262 nm (ε = 36093)
282 nm (ε = 17853)
290 nm (ε = 19404)
300 nm (ε = 15677).

### Example 28

### 3-{3'-[Allylamino]-2'-[hydroxy]-propoxyimino}-2,3-di-{hydro}-1H-benz[e]indene

a) One proceeds according to Example 15.
b) One proceeds according to point b) of Example 16 except that instead of N-(1-methylethyl)-prop-2-ylamine allylamine (6.28 g, 0.11 mole) is used.
Yield: 20.84 g (79%), m.p.: 74-76°C (n-hexane).
Hydrochloride (1/1), m.p.: 188-197 °C (ethanol).

| Analysis for the formula C₁₉H₂₃N₂Cl0₂ (346,87): | | | | |
|---|---|---|---|---|
| Calculated: | C%=65.79, | H%=6.68, | Cl%=10.22, | N%=8.08; |
| Found: | C%=65.30, | H%=6.73, | Cl%=10.20, | N%=8.37. |

- UV: λₘₐₓ=: 243 nm (ε = 30817)
253 nm (ε = 38826)
262 nm (ε = 33850)
281 nm (ε = 13200)
290 nm (ε = 15532)
302 nm (ε = 12690).

### Example 29

### (±)-3-{3'-[Propylamino-2'-[hydroxy]-propoxyimino}-2,3-di-{hydro}-1H-benz[e]indene

a) One proceeds according to Example 15.
b) The product obtained according to Example 15 (21.61 g, 0.085 mole) is reacted with propylamine (60.31 g, 1.02 mole) in a closed pressure-tight flask at a temperature of 90-100°C (on an oil bath) for 12 hours. The excess of amine is distilled off and the product is purified by acidic-alkaline precipitation.
Yield: 22.04 g (83%), m.p.: 80-81°C (n-hexane-ethyl acetate = 1:1).
Hydrochloride (1/1), m.p.: 217-220 °C (ethyl acetate).

| Analysis for the formula C₁₉H₂₅N₂Cl0₃ (348.88): | | | | |
|---|---|---|---|---|
| Calculated: | C%=65.42, | H%=7.22, | Cl%=10.16, | N%=8.03; |
| Found: | C%=65.61, | H%=7.13, | Cl%=10.18, | N%=8.01. |

- UV: λₘₐₓ=: 143 nm (ε = 31155)
253 nm (ε = 38479)
262 nm (ε = 34045)
280 nm (ε = 13477)
290 nm (ε = 15919)
302 nm (ε = 12735).

### Example 30

### 3-{3'-[1''-(Methyl)-ethylamino]-2'-[hydroxy]-propoxyimino]-2,3-di-{hydro}-1H-benz[e]indene

a) One proceeds according to Example 15.
b) One proceeds according to point b) of Example 29 except that instead of propylamine isopropylamine (60.31 g, 1.02 mole) is used.
Yield: 22.3 g (84%), m.p.: 107-108°C (cyclohexane).
Hydrochloride (1/1), m.p.: 210-216 °C (ethanol).

| Analysis for the formula C₁₉H₂₅ClN₂0₂ (348.88): | | | | |
|---|---|---|---|---|
| Calculated: | C%=65.41, | H%=7.22, | Cl%=10.16, | N%=8.03; |
| Found: | C%=65.73, | H%=6.98, | Cl%= 9.92, | N%=8.18. |

- UV: λₘₐₓ=: 244 nm (ε = 3400)
252 nm (ε = 41405)
263 nm (ε = 37856)
282 nm (ε = 14375)
291 nm (ε = 16564)
303 nm (ε = 1775).

### Example 31

### 3-{3'-[1'',1''-Di-(methyl)-ethylamino]-2-[hydroxy]-propoxyimino}-2,3-di-{hydro}-1H-benz[e]indene

a) One proceeds according to Example 15.
b) One proceeds according to point b) of Example 29 except that instead of propylamine tert.-butylamine (74.60 g, 1.02 mole) is used.
Yield: 23.72 g (85.5%), m.p.: 128-129°C (isopropanol).
Hydrochloride (1/1), m.p.: 226-227 °C (isopropanol).

| Analysis for the formula C₂₀H₂₇ClN₂0₂ (362.92): | | | | |
|---|---|---|---|---|
| Calculated: | C%=66.19, | H%=7.50, | Cl%=9.77, | N%=7.72; |
| Found: | C%=66.57, | H%=7.68, | Cl%=9.82, | N%=7.82. |

- UV: λₘₐₓ=: 244 nm (ε = 31879)
254 nm (ε = 40024)
263 nm (ε = 35600)
281 nm (ε = 16520)
291 nm (ε = 16520)
300 nm (ε = 13264).

### Example 32

### 3-{3'-[(1'',1''-Di-〈methyl〉-propyn-2''-yl)-amino]-2-[hydroxy]-propoxyimino}-2,3-di-{hydro}-1H-benz[e]indene

a) One proceeds according to Example 15.
b) One proceeds according to point b) of Example 29 except that instead of propylamine 1,1-dimethylpropyn-2-yl amine (84.8 g, 1.02 mole) is used.
Yield: 23.67 g (82.8%), m.p.: 113-114°C (isopropanol).
Hydrochloride (1/1), m.p.: 220-224 °C (ethanol).

| Analysis for the formula C₂₁H₂₅ClN₂0₂ (372.90): | | | | |
|---|---|---|---|---|
| Calculated: | C%=67.64, | H%=6.76, | Cl%=9.51, | N%=7.51; |
| Found: | C%=67.88, | H%=6.67, | Cl%=9.42, | N%=7.40. |

- UV: λₘₐₓ=: 245 nm (ε = 30978)
252 nm (ε = 39669)
263 nm (ε = 34338)
282 nm (ε = 13204)
291 nm (ε = 15094)
303 nm (ε = 12137).

### Example 33

### 7-[Methyl]-3-[1'-(2',3'-epoxy)-propoxyimino]-2,3-di-[hydro]-1H-benz[e]indene

One proceeds according to Example 15 except that instead of 2,3-dihydro-1H-benz[e]inden-3-one oxime 7-methyl-(2,3-dihydro-1H-benz[e]inden-3-one oxime (21.12 g, 0.1 mole) is used.
Yield: 22.75 g (85%), m.p.: 147-148°C (dioxane).

### Example 34

### 3-{3'-[Propylamino]-2'-[hydroxy]-propoxyimino}-7-{methyl}-2,3-di-{hydro}-1H-benz[e]indene

a) One proceeds according to Example 33.
b) The product obtained according to point a) (22.75 g, 0.085 mole) is reacted with propylamine (60.31 g, 1.02 mole) in a closed, pressure-tight flask at a temperature of 90-100°C (on an oil bath) for 12 hours. The excess of amine is distilled off and the product is purified by acidic-alkaline precipitation.
Yield: 23.15 g (83.5%), m.p.: 98-99°C .
Hydrochloride (1/1), m.p.: 213-215 °C.

| Analysis for the formula C₂₀H₂₇N₂Cl0₂ (362.91): | | | | |
|---|---|---|---|---|
| Calculated: | C%=66.19, | H%=7.50, | Cl%=9.77, | N%=7.72; |
| Found: | C%=66.55, | H%=7.56, | Cl%=9.85, | N%=7.85. |

- UV: λₘₐₓ=: 246 nm (ε = 33579)
253 nm (ε = 41425)
260 nm (ε = 36971)
283 nm (ε = 14124)
292 nm (ε = 16196)
303 nm (ε = 14193).

### Example 35

### 3-{3'-[1''-(Methyl)-ethylamino]-2'-[hydroxy]-propoxyimino]-7-{methyl}-2,3-di-{hydro}-1H-benz[e]indene

a) One proceeds according to Example 33.
b) One proceeds according to point b) of Example 34 except that instead of propylamine isopropylamine (60.31 g, 1.02 mole) is used.
Yield: 23.44 g (84.5%), m.p.: 121-122°C (isopropanol).
Hydrochloride (1/1), m.p.: 208-212 °C (water).

| Analysis for the formula C₂₀H₂₇ClN₂0₂ (362.91): | | | | |
|---|---|---|---|---|
| Calculated: | C%=66.19, | H%=7.50, | Cl%=9.77, | N%=7.72; |
| Found: | C%=65.74, | H%=7.81, | Cl%=9.70, | N%=7.50. |

- UV: λₘₐₓ=: 246 nm (ε = 34801)
252 nm (ε = 43800)
260 nm (ε = 37498)
283 nm (ε = 15602)
292 nm (ε = 17101)
302 nm (ε = 14161).

### Example 36

### 3-[3'-(Cyclopropylamino)-2'-(hydroxy)-propoxyimino]-7-[methyl]-2,3-di-[hydro]-1H-benz[e]indene

a) One proceeds according to Example 33.
b) One proceeds according to point b) of Example 34 except that instead of propylamine cyclopropylamine (58.24 g, 1.02 mole) is used.
Yield: 23.44 g (85%), m.p.: 118-119°C (isopropanol).
Hydrochloride (1/1), m.p.: 178-181 °C (isopropanol).

| Analysis for the formula C₂₀H₂₅ClN₂0₂ (360.89): | | | | |
|---|---|---|---|---|
| Calculated: | C%=66.56, | H%=6.98, | Cl%=9.83, | N%=7.76; |
| Found: | C%=66.04, | H%=6.99, | Cl%=9.82, | N%=7.60. |

- UV: λₘₐₓ=: 246 nm (ε = 40046)
252 nm (ε = 44110)
262 nm (ε = 38849)
292 nm (ε = 17392)
304 nm (ε = 14685).

### Example 37

### 3-{3'-[N-(2''-〈Dimethylamino〉-ethyl)-amino]-2'-[hydroxy]-propoxyimino}-7-{methyl}-2,3-di-{hydro}-1H-benz[e]indene

a) One proceeds according to Example 33.
b) One proceeds according to point b) of Example 34 except that instead of propylamine 2-dimethylaminoethylamine (89.91 g, 1.02 mole) is used and the reaction is carried out at a temperature of 140°C.
Yield: 26.36 g (87%), m.p.: 123-124°C (isopropanol).
Hydrochloride (1/2), m.p.: 209-213 °C (isopropanol).

| Analysis for the formula C₂₁H₃₂Cl₂N₃0₂ (429.43): | | | | |
|---|---|---|---|---|
| Calculated: | C%=58.74, | H%=7.51, | Cl%=16.51, | N%=9.79; |
| Found: | C%=58.97, | H%=7.36, | Cl%=16.30, | N%=9.30. |

- UV: λₘₐₓ=: 254 nm (ε = 42210)
261 nm (ε = 36088)
293 nm (ε = 13708)
303 nm (ε = 12646).

### Example 38

### 3-{3'-[N-3''-〈Dimethylamino〉-propyl)-amino]-2'-[hydroxy]-propoxyimino}-7-{methyl}-2,3-di-{hydro}-1H-benz[e]indene

a) One proceeds according to Example 33.
b) One proceeds according to point b) of Example 34 except that instead of propylamine 3-dimethylamino-1-propylamine (104.22 g, 1.02 mole) is used and the reaction is carried out at 150°C.
Yield: 26.92 g (85.5%), m.p.: 123-124°C (isopropanol).
Hydrochloride (1/2), m.p.: 209-213 °C (ethanol).

| Analysis for the formula C₂₂H₃₄Cl₂N₃0₂ (443.45): | | | | |
|---|---|---|---|---|
| Calculated: | C%=58.74, | H%=7.51, | Cl%=16.51, | N%=9.79; |
| Found: | C%=58.97, | H%=7.36, | Cl%=16.30, | N%=9.30. |

- UV: λₘₐₓ=: 253 nm (ε = 43466)
260 nm (ε = 34894)
291 nm (ε = 15187)
302 nm (ε = 12672).

### Example 39

### 3-{3'-[4''-(Pyrid-2'''-yl)-piperazin-1''-yl]-2'-[hydroxy]-propoxyimino}-7-{methyl}-2,3-di-{hydro}-1H-benz[e]indene

a) One proceeds according to Example 33.
b) One proceeds according to point b) of Example 27 except that the compound obtained according to Example 33 is further reacted.
Yield: 31.1 g (84.9%), m.p.: 149-150°C (acetonitrile).
Hydrochloride (1/2), m.p.: 177-182 °C (ethanol).

| Analysis for the formula C₂₆H₃₂Cl₂N₄0₂ (503.49): | | | | |
|---|---|---|---|---|
| Calculated: | C%=62.02, | H%=6.41, | Cl%=14.08, | N%=11.13; |
| Found: | C%=64.10, | H%=6.69, | Cl%=13.63, | N%=10.89. |

- UV: λₘₐₓ=: 242 nm (ε = 44821)
252 nm (ε = 45555)
260 nm (ε = 38740)
283 nm (ε = 17037)
291 nm (ε = 20182)
301 nm (ε = 16513).

### Example 40

### Tablet comprising 25 mg of active ingredient

The composition of one tablet is as follows:

| | |
|---|---|
| active ingredient | 25.0 mg |
| corn starch | 97.0 mg |
| polyvinyl-pyrrolidone | 175.0 mg |
| magnesium stearate | 3.0 mg |
| | $\overline{\text{300.0 mg}}$ |

The tablet is prepared as follows:
The active ingredient and the corn starch are admixed, then wetted with an aqueous polyvinyl-pyrrolidone solution having a concentration of 10 to 15% by weight and the mixture is granuled, then dried at a temperature of 40 to 50°C. The dry granules are rubbed through a sieve, mixed with talc and magnesium stearate and tablets are prepared from the mixture.

The weight of one tablet is 300.0 mg.

### Example 41

### Tablet comprising 250 mg of active ingredient

The composition of one tablet is as follows:

| | |
|---|---|
| active ingredient | 250.0 mg |
| lactose | 270.0 mg |
| corn starch | 75.0 mg |
| magnesium stearate | 5.0 mg |
| | $\overline{\text{600.0 mg}}$ |

The active ingredient, the lactose and the corn starch are wetted and mixed, granulated and dried at a temperature of 40 to 50°C. The dry granules are rubbed through a sieve as described hereinabove, mixed with magnesium stearate and talc, then tablets are formed.

The weight of one tablet is 600.0 mg.

### Example 42

### Dragées comprising 25 mg of active ingredient

The composition of one dragée core is as follows:

| | |
|---|---|
| active ingredient | 25.0 mg |
| corn starch | 245.0 mg |
| talc | 18.0 mg |
| gelatin | 8.0 |
| magnesium stearate | 4.0 mg |
| | $\overline{\text{300.0 mg}}$ |

The tablet is prepared as follows:
The active ingredient and the corn starch are mixed, wetted with an aqueous gelatin solution having a concentration of 10% by weight, granules are formed from the wet mixture, then the granules are dried at a temperature of 40 to 50°C. The dry granules are rubbed through a sieve, homogenized with talc and magnesium stearate and dragée cores of 300.0 mg are compressed from the mixture.

### Example 43

### Dragées comprising 50.0 mg of active ingredient

The composition of one dragée core is as follows:

| | |
|---|---|
| active ingredient | 50.0 mg |
| lactose | 97.0 mg |
| polyvinyl-pyrrolidone | 2.0 mg |
| magnesium stearate | 1.0 mg |

The method of preparation of the granules is as described in Example 42 and the concentration of the polyvinyl-pyrrolidone solution is identical to that in Example 40. The weight of the dragée core is 150.0 mg.

The dragée cores are coated with a layer containing sugar and talc in a manner known per se. The dragées thus obtained are painted with non-toxic food paint to the desired colour and polished with bees-wax.

### Example 44

### Gelatin capsule comprising 5.0 mg of active ingredient

The composition of one gelatin capsule is as follows:

| | |
|---|---|
| active ingredient | 5.0 mg |
| corn starch | 40.0 mg |
| siliciumdioxide (aerosil®) | 3.0 mg |
| magnesium stearate | 2.0 mg |
| | $\overline{\text{50.0 mg}}$ |

The ingredients are homogenized and filled into gelatin capsules of suitable size.

### Example 45

### Gelatin capsule comprising 25.0 mg of active ingredient

The composition of one gelatin capsule is as follows:

| | |
|---|---|
| active ingredient | 25.0 mg |
| corn starch | 265.0 mg |
| siliciumdioxide (aerosil®) | 6.0 mg |
| magnesium stearate | 4.0 mg |
| | $\overline{\text{300.0 mg}}$ |

The ingredients are homogenized and filled into gelatin capsules of suitable size.

### Example 46

### Gelatin capsule comprising 50.0 mg of active ingredient

The composition of one gelatin capsule is as follows:

| | |
|---|---|
| active ingredient | 50.0 mg |
| lactose | 90.0 mg |
| siliciumdioxide (aerosil®) | 6.0 mg |
| magnesium stearate | 4.0 mg |
| | $\overline{\text{150.0 mg}}$ |

The ingredients are homogenized and filled into gelatin capsules of suitable size.

### Example 47

### Gelatin capsule comprising 250.0 mg of active ingredient

The composition of one gelatin capsule is as follows:

| | |
|---|---|
| active ingredient | 250.0 mg |
| lactose | 148.0 mg |
| magnesium stearate | 2.0 mg |
| | $\overline{\text{400.0 mg}}$ |

The ingredients are homogenized and filled into gelatin capsules of suitable size.

### Example 48

### Injection solution comprising 25.0 mg of active ingredient

The composition of one ampoule is as follows:

| | |
|---|---|
| active ingredient | 25.0 mg |
| sodium chloride | 5.0 mg |
| dissolved in 5 cm³ of twice-distilled water. | |

The active ingredient and sodium chloride are dissolved in the necessary amount of twice-distilled water suitable for making injection solutions. The solution is filtered, filled into ampoules and sterilized.

### Example 49

### Injection solution comprising 50.0 mg of active ingredient

The composition of one ampoule is as follows:

| | |
|---|---|
| active ingredient | 50.0 mg |
| sodium chloride | 10.0 mg |

The active ingredient and the sodium chloride are dissolved in the necessary amount of twice-distilled water, then filled into ampoules under sterile conditions.

### Example 50

### Suppository comprising 250 mg of active ingredient

The composition of one suppository is as follows:

| | |
|---|---|
| active ingredient | 250.0 mg |
| fatty acid glyceride | 750.0 mg |

The fatty acid glyceride is melted, the active ingredient is homogenized in the melt, then the melt is poured into a mould. One suppository weighs 1000.0 mg and comprises 250.0 mg of active ingredient.

### Example 51

### Drop comprising 5% by weight of active ingredient

| | |
|---|---|
| active ingredient | 25.0 mg |
| sorbitol | 340.0 mg |
| polyethylene glycol | 100.0 mg |
| citric acid | 1.0 mg |
| sodium citrate | 3.0 mg |
| ion-free water | 30.0 cm³ |
| flavourant | 1.0 mg |
| | $\overline{\text{500.0 mg}}$ |

The sorbitol, the active ingredient, citric acid and sodium citrate are dissolved in the aqueous solution of polyethylene glycol, then after dissolution of the solid material the flavourant is added. The solution is filtered and filled into flasks supplied with a drop-dispenser.

## Claims

1. Benz[e]indene derivatives of the general formula 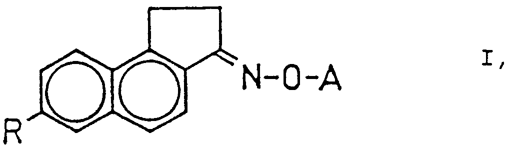 wherein
A represents a group of the formula 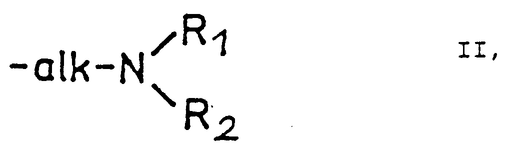 in which latter
alk means a C₂₋₇ alkylene group, optionally substituted by a hydroxy group and
R₁ and R₂ stand independently for hydrogen or a C₁₋₇ alkyl, C₂₋₇ alkenyl, C₂₋₇ alkynyl, C₃₋₇ cycloalkyl, mono-(C₁₋₇)alkylamino-(C₁₋₇)alkyl or di-(C₁₋₇)alkylamino-(C₁₋₇)alkyl group or
R₁ and R₂ together with the nitrogen atom to which they are attached form a 4 to 7 membered heterocyclic ring, optionally comprising an oxygen atom or a further nitrogen atom, which latter may be substituted by a phenyl, benzyl, pyridyl, pyrimidinyl or C₁₋₃ alkyl group which latter ones, in turn, may be substituted by a hydroxy or methoxy group or a halogen atom or a halophenyl group or
R₁ and R₂ together with the nitrogen atom to which they are attached form a phthalimido group or
A means a pyrimidino group, a 2,3-(epoxy)-propyl group or a group of the formula 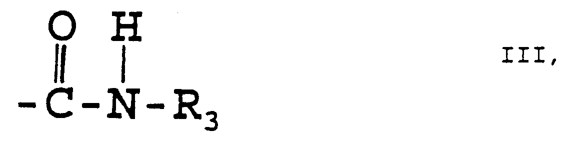 in which latter
R₃ stands for a C₁₋₇ alkyl, C₂₋₇ alkenyl or C₃₋₈ cycloalkyl group and
R denotes hydrogen or a C₁₋₇ alkyl group,
and their stereoisomers and optically active isomers and mixtures thereof as well as acid addition salts and quaternary ammonium derivatives of these compounds.

2. Benz[e]indene derivatives according to claim 1, characterized in that the alkyl group(s) which may be repre sented by R₁ and/or R₂ and/or R₃ and/or R including those occuring in mono-(C₁₋₇)alkylamino-(C₁₋₇)alkyl or di-(C₁₋₇)alkylamino-(C₁₋₇)alkyl groups which may be represented by R₁ and/or R₂ is/are such having from 1 to 4, particularly 1 to 3 or 6 carbon atom(s) and/or the alkyl group which may be carried by the heterocyclic ring which may be formed by R₁ and R₂ together with the nitrogen atom to which they are attached is such having from 1 or 2 carbon atom(s).

3. Benz[e]indene derivatives according to claim 1 or 2, characterized in that the alkenyl group(s) which may be represented by R₁ and/or R₂ or R₃ and/or the alkylene group which may be represented by alk is/are such having from 2 to 4, particularly 2 or 3 carbon atom(s) and/or the alkynyl group(s) which may be represented by R₁ and/or R₂ is/are such having from 2 to 5 carbon atom(s) and/or the cycloalkyl group(s) which may be represented by R₁ and/or R₂ is/are such having from 3 to 6, particularly 3 or 6, carbon atom(s) and/or the cycloalkyl group which may be represented by R₃ is such having from 5 or 6, particularly 6, carbon atom(s).

4. Benz[e]indene derivatives according to claim 1 or 2, characterized in that the heterocyclic ring which may be formed by R₁ and R₂ together with the nitrogen atom to which they are attached is such having from 5 to 7, particularly 5 or 6, members.

5. Benz[e]indene derivatives according to claims 1 to 4, characterized in that the halogen atom or the halogen atom of the halophenyl group by which the phenyl, benzyl, pyrimidinyl or alkyl group which may be carried by the heterocyclic ring which may be formed by R₁ and R₂ together with the nitrogen atom to which they are attached may be substituted is chlorine or fluorine.

6. 3-[3'-(Cyclopropylamino)-2'-(hydroxy)-propoxyimino]-2,3-di-[hydro]-1H-benz[e]indene and its acid addition salts and quaternary ammonium derivatives.

7. Process for preparing the benz[e]indene derivatives according to claims 1 to 6, characterized in that in a manner known per se
a) for preparing benz[e]indene derivatives of the formula I, wherein A stands for a 2,3-(epoxy)-propyl group reacting benz[e]indene derivatives of the formula 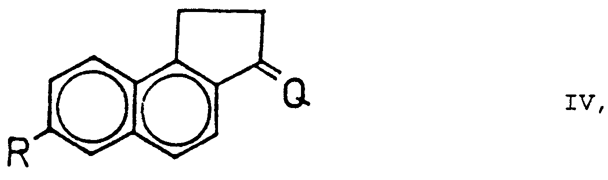 wherein Q represents a group of the formula =N-OH and R is as defined in claim 1 or 2,with halo-epoxypropyl compounds of the formula 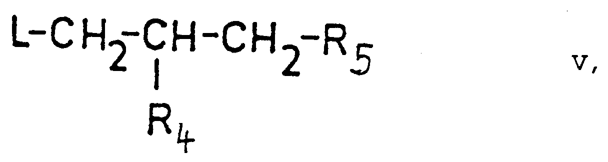 wherein L stands for halogen and R₄ and R₅ together represent oxygen, in the presence of basic condensing agents or
b) for preparing benz[e]indene derivatives of the formula I, wherein A denotes a group of the formula II, wherein alk, R₁ and R₂ are as defined in claims 1 to 5,
b₁) reacting benz[e]indene derivatives of the formula IV, wherein Q stands for a group of the formula =N-OH and R is as defined in claim 1 or 2, or acid addition salts thereof with halo compounds of the formula 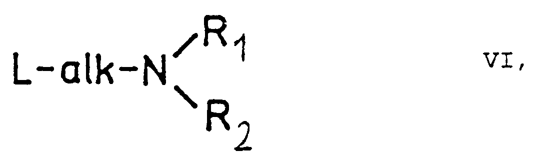 wherein alk, R₁ and R₂ are as defined in claims 1 to 5 and L stands for halogen, or with acid addition salts thereof in the presence of basic condensing agents or
b₂) reacting benz[e]indene derivatives of the formula I, wherein A stands for a 2,3-(epoxy)-propyl group, with amines of the formula 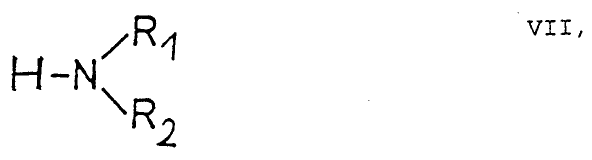 wherein R₁ and R₂ are as defined in claims 1 to 5, or
b₃) reacting benz[e]indene derivatives of the formula IV, wherein Q stands for oxygen or sulphur and R is as stated above, with, optionally substituted, aminoalkyl halogenides of the formula VI, wherein alk, R₁ and R₂ are as defined in claims 1 to 5 and L represents a group of the formula H₂N-0-, or with acid addition salts thereof in the presence of basic condensing agents or
c) for preparing benz[e]indene derivatives of the formula I, wherein A stands for a pyrimidino group reacting benz[e]indene derivatives of the formula IV, wherein Q represents a group of the formula =N-OH and R is as defined in claim 1 or 2, with halopyrimidines of the formula 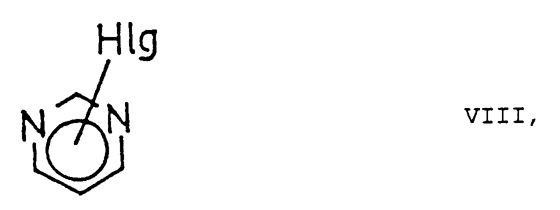 wherein Hlg represents halogen, in the presence of basic condensing agents or
d) for preparing benz[e]indene derivatives of the formula I, wherein A stands for a group of the formula III, reacting benz[e]indene derivatives of the formula IV, wherein Q is a group of the formula =N-OH and R is as defined in claim 1 or 2, with isocyanates of the formula
R₃-N=C=0 IX,
wherein R₃ is as defined in claims 1 to 3,
and, if desired, converting benz[e]indene derivatives of the formula I thus obtained into acid addition salts or quaternary ammonium derivatives or, if desired, liberating free benz[e]indene derivative bases of the formula I from salts thereof and, if desired, converting the free benz[e]indene derivative bases into other acid addition salts or quaternary ammonium derivatives and/or, if desired, separating the stereoisomers and/or optically active isomers or racemizing the latter ones.

8. Medicaments, characterized by that they contain as active principle(s) at least one compound according to claims 1 to 6, suitably in admixture with at least one inert solid and/or liquid pharmaceutical carrier and/or diluent and/or further auxiliary excipient.

9. Use of the compounds according to claims 1 to 6, for the preparation of medicaments, particularly such having tranquillo-sedative, anticonvulsive and antianginal effects.

## Patentansprüche

1. Benz[e]inden-Derivate der allgemeinen Formel 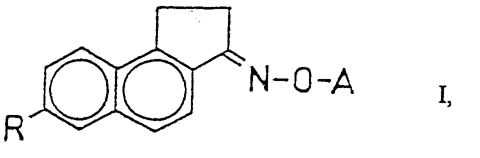 worin
A eine Gruppe der Formel 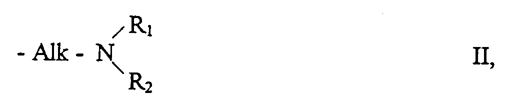 repräsentiert, wobei in letzterer
Alk eine C₂₋₇-Alkylengruppe, gegebenenfalls durch eine Hydroxygruppe substituiert, bedeutet, und
R₁ und R₂ unabhängig voneinander Wasserstoff oder eine C₁₋₇-Alkyl-, C₂₋₇-Alkenyl-, C₂₋₇-Alkynyl-, C₃₋₇-Cycloalkyl-, Mono-(C₁₋₇)alkylamino-(C₁₋₇)alkyl- oder Di-(C₁₋₇)alkyl-amino-(C₁₋₇)-alkylgruppe bedeuten, oder
R₁ und R₂ zusammen mit dem Stickstoffatom, an dem sie gebunden sind, einen 4- bis 7-gliedrigen heterocyclischen Ring bilden, der gegebenenfalls ein Sauerstoffatom oder ein weiteres Stickstoffatom beinhaltet, wobei letzteres durch eine Phenyl-, Benzyl-, Pyridyl-, Pyrimidinyl- oder C₁₋₃-Alkylgruppe substituiert sein kann, wobei letztere ihrerseits durch eine Hydroxy- oder Methoxygruppe oder ein Halogenatom oder eine Halogenphenylgruppe substituiert sein können, oder
R₁ und R₂ zusammen mit dem Stickstoffatom, an dem sie gebunden sind, eine Phthalimidogruppe bilden, oder
A eine Pyrimidinogruppe, eine 2,3-(Epoxy)-propylgruppe oder eine Gruppe der Formel 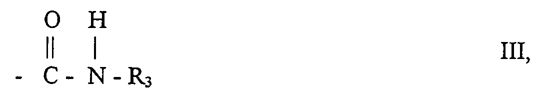 bedeutet, in welcher
R₃ für eine C₁₋₇-Alkyl-, C₂₋₇-Alkenyl- oder C₃₋₈-Cycloalkylgruppe steht, und
R Wasserstoff oder eine C₁₋₇-Alkylgruppe bedeutet,
und deren Stereoisomere und optisch aktiven Isomere und Mischungen davon sowie Säureadditionssalze und quaternäre Ammonium-Derivate dieser Verbindungen.

2. Benz[e]inden-Derivate gemäß Anspruch 1, dadurch gekennzeichnet, daß die Alkylgruppe bzw. Alkylgruppen, welche durch R₁ und/oder R₂ und/oder R₃ und/oder R, einschließlich jenen in Mono-(C₁₋₇)alkylamino-(C₁₋₇)alkyl- oder Di-(C₁₋₇)alkylamino-(C₁₋₇)alkylgruppen auftretenden, welche durch R₁ und/oder R₂ repräsentiert werden können, solche mit 1 bis 4, insbesondere 1 bis 3, oder 6 Kohlenstoffatomen ist/sind, und/oder die Alkylgruppe, welche von dem heterocyclischen Ring getragen werden kann, welcher durch R₁ und R₂ zusammen mit dem Stickstoffatom, an dem sie gebunden sind, gebildet sein kann, eine solche mit 1 oder 2 Kohlenstoffatomen ist.

3. Benz[e]inden-Derivate gemäß Anspruch 1 oder 2, dadurch gekennzeichnet, daß die Alkenylgruppe bzw. Alkenylgruppen, welche durch R₁ und/oder R₂ oder R₃ repräsentiert werden kann/können, und/oder die Alkylengruppe, welche durch Alk repräsentiert werden kann, solche ist/sind, welche 2 bis 4, insbesondere 2 oder 3, Kohlenstoffatome besitzen, und/oder die Alkinylgruppe bzw. Alkinylgruppen, welche durch R₁ und/oder R₂ repräsentiert werden kann/können, solche mit 2 bis 5 Kohlenstoffatomen ist/sind, und/oder die Cycloalkylgruppe bzw. Cycloalkylgruppen, welche durch R₁ und/oder R₂ repräsentiert werden kann/können, solche mit 3 bis 6, insbesondere 3 oder 6, Kohlenstoffatomen ist/sind, und/oder die Cycloalkylgruppe, welche durch R₃ repräsentiert werden kann, eine solche mit 5 oder 6, insbesondere 6, Kohlenstoffatomen ist.

4. Benz[e]inden-Derivate gemäß Anspruch 1 oder 2, dadurch gekennzeichnet, daß der heterocylclische Ring, welcher durch R₁ und R₂ zusammen mit dem Stickstoffatom, an dem sie gebunden sind, gebildet werden kann, ein solcher mit 5 bis 7, insbesondere 5 oder 6, Gliedern ist.

5. Benz[e]inden-Derivate gemäß Anspruch 1 bis 4, dadurch gekennzeichnet, daß das Halgenatom oder das Halogenatom der Halogenphenylgruppe, durch welches die Phenyl-, Benzyl-, Pyrimidinyl- oder Alkylgruppe, welche durch den heterocyclischen Ring, welcher durch R₁ und R₂ zusammen mit dem Stickstoffatom, an dem sie gebunden sind, gebildet sein kann, substituiert sein kann, Chlor oder Fluor ist.

6. 3-[3'-(Cyclopropylamino)-2'-(hydroxy)-propoxyimino]-2,3-di-[hydro]-1H-benz[e]inden und seine Säureadditionsalze und quaternären Ammonium-Derivate.

7. Verfahren zur Herstellung der Benz[e]inden-Derivate gemäß Anspruch 1 bis 6, dadurch gekennzeichnet, daß in einer per se bekannten Weise
a) zur Herstellung von Benz[e]inden-Derivaten der Formel I, worin A für eine 2,3-(Epoxy)-propylgruppe steht, Benz[e]inden-Derivate der Formel 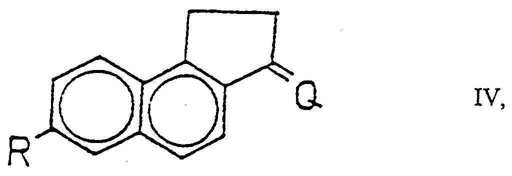 worin Q für eine Gruppe der Formel =N-OH steht und die Bedeutung von R die gleiche wie in Anspruch 1 oder 2 ist, mit Halogenepoxypropylverbindungen der Formel 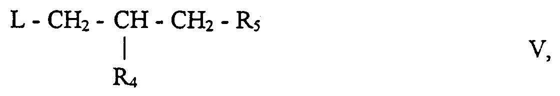 worin L für Halogen steht und R₄ und R₅ zusammen für Sauerstoff stehen, in Gegenwart von basischen Kondensationsmitteln umgesetzt werden, oder
b) zur Herstellung von Benz[e]inden-Derivaten der Formel I, worin A für eine Gruppe der Formel II steht, worin die Bedeutung von Alk, R₁ und R₂ die gleiche wie in den Ansprüchen 1 bis 5 ist,
b₁) Benz[e]inden-Derivate der Formel IV, worin Q für eine Gruppe der Formel =N-OH steht und die Bedeutung von R die gleiche wie in Anspruch 1 und 2 ist, oder Säureadditionssalze davon mit Halogenverbindungen der Formel 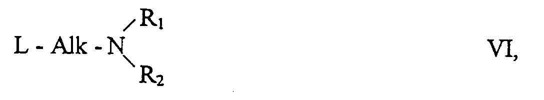 worin die Bedeutung von Alk, R₁ und R₂ die gleiche wie in den Ansprüchen 1 bis 5 ist und L für Halogen steht, oder deren Säureadditionssalze in Gegenwart von basischen Kondensationsmitteln umgesetzt werden, oder
b₂) Benz[e]inden-Derivate der Formel I, worin A für eine 2,3-(Epoxy)-propylgruppe steht, mit Aminen der Formel 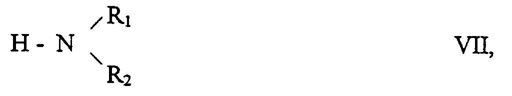 worin die Bedeutung von R₁ und R₂ die gleiche wie in den Ansprüchen 1 bis 5 ist, umgesetzt werden, oder
b₃) Benz[e]inden-Derivate der Formel IV, worin Q für Sauerstoff oder Schwefel steht und die Bedeutung von R die gleiche wie oben ist, mit gegebenenfalls substituierten Aminoalkylhalogeniden der Formel VI, worin die Bedeutung von Alk, R₁ und R₂ die gleiche wie in den Ansprüchen 1 bis 5 ist und L für eine Gruppe der Formel H₂N-O- steht, oder deren Säureadditionssalze in Gegenwart von basischen Kondensationsmitteln umgesetzt werden, oder
c) zur Herstellung von Benz[e]inden-Derivaten der Formel I, worin A für eine Pyrimidinogruppe steht, Benz[e]inden-Derivate der Formel IV, worin Q für eine Gruppe der Formel =N-OH steht und die Bedeutung von R die gleiche wie im Anspruch 1 oder 2 ist, mit Halogenpyrimidinen der Formel 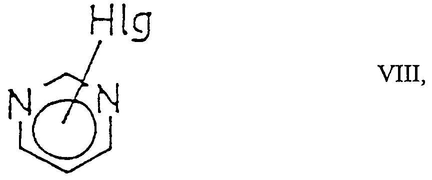 worin Hlg für Halogen steht, in Gegenwart von basischen Kondensationsmitteln umgesetzt werden, oder
d) zur Herstellung von Benz[e]inden-Derivaten der Formel I, worin A für eine Gruppe der Formel III steht, Benz[e]inden-Derivate der Formel IV, worin Q eine Gruppe der Formel =N-OH ist und die Bedeutung von R die gleiche wie im Anspruch 1 oder 2 ist, mit Isocyanaten der Formel
R₃-N=C=O IX,
worin die Bedeutung von R₃ die gleiche wie in den Ansprüchen 1 bis 3 ist, umgesetzt werden,
und gewünschtenfalls die derart erhaltenen Benz[e]inden-Derivate der Formel I zu Säureadditionssalzen oder quaternären Ammonium-Derivaten umgewandelt werden, oder, falls gewünscht, freie Basen der Benz[e]inden-Derivate der Formel I aus deren Salzen freigesetzt werden, und, falls gewünscht, die freien Basen der Benz[e]inden-Derivate zu anderen Säureadditionssalzen oder quaternären Ammonium-Derivaten umgewandelt werden, und/oder, falls gewünscht, die Stereoisomeren und/oder optisch aktiven Isomeren getrennt oder die letzteren racemisiert werden.

8. Medikamente, welche dadurch gekennzeichnet sind, daß sie als aktiven Hauptbestandteil bzw. als aktive Hauptbestandteile mindestens eine Verbindung gemäß Anspruch 1 bis 6 enthalten, geeigneterweise in Vermischung mit mindestens einem inerten festen und/oder flüssigen pharmazeutischen Träger und/oder Verdünnungsmittel und/oder weiteren Hilfsstoffen.

9. Verwendung der Verbindungen gemäß den Ansprüchen 1 bis 6 zur Herstellung von Medikamenten, insbesondere jenen mit tranquillo-sedativen, krampfhemmenden und antianginalen Wirkungen.

## Revendications

1. Dérivés de benz[e]indène de la formule générale 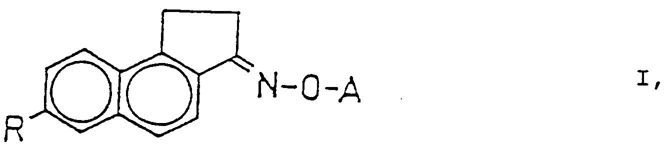 dans laquelle
A représente un groupe de la formule 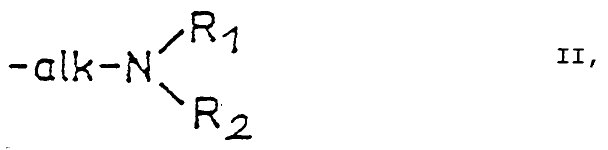 dans cette dernière
alk signifie un groupe C₂₋₇ alkylénique facultativement substitué par un groupe hydroxy et
R₁ et R₂ représentent indépendamment de l'hydrogène ou un groupe C₁₋₇alkylique, C₂₋₇alkénylique, C₂₋₇ alkynylique, C₃₋ ₇cycloalkylique, mono-(C₁₋₇)alkylamino-(C₁₋₇)alkylique ou di-(C₁₋₇)alkylamino-(C₁₋₇)alkylique ou
R₁ et R₂ ensemble avec l'atome d'azote auquel ils sont attachés forment un anneau hétérocyclique de 4 à 7 membres, comportant facultativement un atome d'oxygène ou un atome d'azote supplémentaire, ce dernier pouvant être substitué par un groupe phénylique, benzylique, pyridylique, pyrimidinylique ou C₁₋₃alkylique, ces derniers groupes pouvant être substitués à leur tour par un groupe hydroxy ou méthoxy ou un atome d'halogène ou un groupe halophénylique ou
R₁ et R₂ ensemble avec l'atome d'azote auquel ils sont attachés forment un groupe phthalimido ou
A signifie un groupe pyrimidino, un groupe 2,3-(époxy)-propylique ou un groupe de la formule 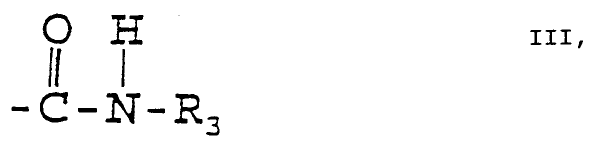 dans cette dernière
R₃ représente un groupe C₁₋₇alkylique, C₂₋ ₇alkénylique ou C₃₋₈cycloalkylique et
R représente de l'hydrogène ou un groupe C₁₋₇alkylique,
et leurs stéréoisomères et isomères optiquement actifs et des mélanges de ces-derniers aussi bien que des sels d'addition acides et des dérivés d'ammonium quaternaire de ces composés.

2. Dérivés de benz[e]indène selon les revendications 1 ou 2, caractérisés en ce que le(s) groupe(s) alkylique(s) qui peut(peuvent) être représenté(s) par R₁ et/ou R₂ et/ou R₃ et/ou R y compris ceux qui apparaissent dans des groupes mono-(C₁₋₇)alkylamino-(C₁₋₇)alkyliques ou di-(C₁₋ ₇)alkylamino-(C₁₋₇)alkyliques qui peuvent être représentés par R₁ et/ou R₂ est/sont celui(ceux) qui a(ont) de 1 à 4, surtout 1 à 3 ou 6 atome(s) de carbone et/ou le groupe alkylique qui peut être porté par l'anneau hétérocyclique, qui lui peut être formé par R₁ et R₂ ensemble avec l'atome d'azote auquel ils sont attachés, est celui qui a 1 ou 2 atome(s) de carbone.

3. Dérivés de benz[e]indène selon les revendications 1 ou 2, caractérisés en ce que le(s) groupe(s) alkénylique(s) qui peut(peuvent) être représenté(s) par R₁ et/ou R₂ ou R₃ et/ou le groupe alkylènique qui peut être représenté par alk est/sont celui(ceux) qui a(ont) de 2 à 4, surtout 1 à 3 atome(s) de carbone et/ou le(s) groupe(s) alkynylique(s) qui peut(peuvent) être représenté(s) par R₁ et/ou R₂ est/sont celui(ceux) qui a(ont) de 2 à 5 atomes de carbone et/ou le(s) groupe(s) de cycloalkylique(s) qui peut(peuvent) être représenté(s) par R₁ et/ou R₂ est/sont celui(ceux) qui a(ont) de 3 à 6, surtout 3 ou 6 atomes de carbone et/ou le groupe cycloalkylique qui peut être représenté par R₃ est celui qui a 5 ou 6, surtout 6 atomes de carbone.

4. Dérivés de benz[e]indène selon les revendications 1 ou 2, caractérisés en ce que l'anneau hétérocyclique qui peut être formé par R₁ et R₂ ensemble avec l'atome d'azote auquel ils sont attachés est celui qui a de 5 à 7, surtout 5 ou 6 membres.

5. Dérivés de benz[e]indène selon les revendications 1 à 4, caractérisés en ce que l'atome d'halogène ou l'atome d'halogène du groupe halophénylique par lequel le groupe phénylique, benzylique, pyrimidinylique ou alkylique qui peut être porté par l'anneau hétérocyclique qui à son tour peut être formé par R₁ et R₂ ensemble avec l'atome d'azote auquel il sont attachés peut être remplacé et du chlore ou du fluor.

6. 3-[3'-(cyclopropylamino)-2'-(hydroxy)-propoxyimino]--2,3-di-[hydro]-1H-benz[e]indène et ses sels d'addition acides et dérivés d'ammonium quaternaires.

7. Procédé pour la préparation de dérivés de benz[e]indène selon les revendications 1 à 6, caractérisé en ce que dans une manière en soi connue :
a) pour la préparation de dérivés de benz[e]indène de la formule I, dans laquelle A représente un groupe 2,3-(époxy)-propylique transformant des dérivés de benz[e]indène de la formule 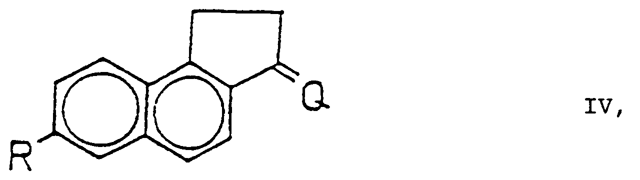 dans laquelle Q représente un groupe de la formule = N-OH et R est défini selon les revendications 1 ou 2, avec des composés halo-époxypropyliques de la formule 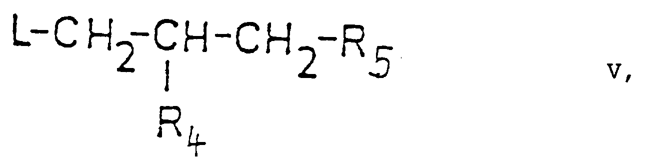 dans laquelle L représente l'halogène et R₄ et R₅ ensemble représentent de l'oxygène, en présence d'agents de condensation basiques ou
b) pour la préparation de dérivés de benz[e]indène de la formule I, dans laquelle A représente un groupe de la formule II, dans laquelle alk, R₁ et R₂ sont définis selon les revendications 1 à 5,
b₁) transformant des dérivés de benz[e]indène de la formule IV, dans laquelle Q représente un groupe de la formule = N-OH et R est défini selon les revendications 1 ou 2, ou des sels d'addition acide de ceux-ci avec des composés halo de la formule 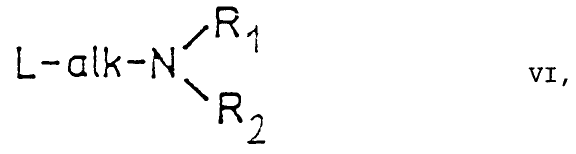 dans laquelle alk, R₁ et R₂ sont définis selon les revendications 1 à 5 et L représente l'halogène ou avec des sels d'addition acides de ceux-ci en présence d'agents de condensation basiques ou
b₂) transformant des dérivés benz[e]indène de la formule I, dans laquelle A représente un groupe 2,3-(époxy)-propylique avec des amines de la formule 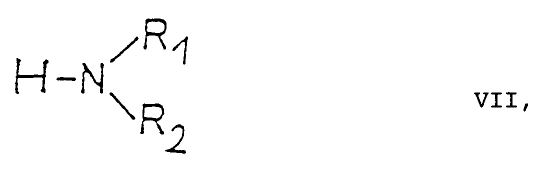 dans laquelle, R₁ et R₂ sont définis selon les revendications 1 à 5, ou
b₃) transformant des dérivés de benz[e]indène de la formule IV, dans laquelle Q représente de l'oxygène ou du soufre et R est défini comme ci-dessus, avec des halogénides aminoalkyliques de la formule VI facultativement substitués, dans laquelle alk, R₁ et R₂ sont définis selon les revendications 1 à 5 et L représente un groupe de la formule H₂N-O-, ou avec des sels d'addition acides de ceux-ci en présence d'agents de condensation basiques ou
c) pour la préparation de dérivés de benz[e]indène de la formule I, dans laquelle A représente un groupe pyrimidino transformant des dérivés de benz[e]indène de la formule IV, dans laquelle Q représente un groupe de la formule =N-OH et R est défini selon les revendications 1 ou 2 avec des halopyrimidines de la formule 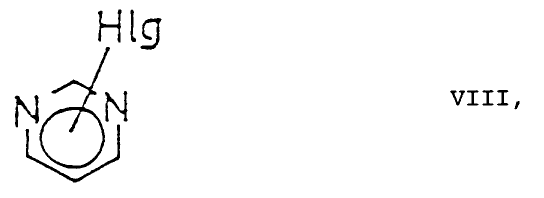 dans laquelle Hlg représente l'halogène en présence d'agents de condensation basiques ou
d) pour la préparation de dérivés de benz[e]indène de la formule I, dans laquelle A représente un groupe de la formule III, transformant des dérivés de benz[e]indène de la formule IV, dans laquelle Q est un groupe de la formule =N-OH et R est défini selon les revendications 1 ou 2, avec des isocyanates de la formule
R₃-N=C=0 IX,
dans laquelle R₃ est défini selon les revendications 1 à 3,
et, s'il est souhaité, convertissant les dérivés de benz[e]indène de la formule I ainsi obtenus en sels d'addition acides ou dérivés d'ammonium quaternaires ou, si souhaité, libérant des bases libres des dérivés de benz[e]indène de la formule I de leurs sels et, si souhaité, transformant des bases libres de dérivés de benz[e]indène en d'autres sels d'addition acides ou d'autres dérivés d'ammonium quaternaires et/ou, si souhaité, séparant les stéréoisomères et/ou isomères optiquement actifs ou racémisant ces derniers.

8. Médicaments, caractérisés par le fait qu'ils contiennent comme principe(s) actif(s) au moins un composé selon les revendications 1 à 6, de préférence dans un mélange avec au moins un support inerte solide et/ou liquide pharmaceutique et/ou diluant et/ou un excipient auxiliaire supplémentaire.

9. Utilisation de composés selon les revendications 1 à 6 pour la préparation de médicaments surtout ceux ayant un effet tranquillisant-sédatif, anticonvulsif et antiangine.
